# EUROPÄISCHE PATENTSCHRIFT

(11) **EP 2 829 292 B1**
(45) Veröffentlichungstag und Bekanntmachung des Hinweises auf die Patenterteilung: **01.11.2017**
(21) Anmeldenummer: 13177387.1
(22) Anmeldetag: 22.07.2013
(51) Int. Cl.: A61M 5/24, A61M 5/315

(54) **Antriebs- und Dosiervorrichtung mit einem Begrenzungselement zur Verhinderung der Einstellung einer höheren Dosis als verfügbar**
Drive and dosing device having a limiting element for preventing the adjustment of a dose higher than available
Dispositif d'entraînement et de dosage comprenant un élément de limitation pour empêcher le réglage d'une dose plus grande que disponible

(43) Veröffentlichungstag der Anmeldung: 28.01.2015
(73) Patentinhaber: TecPharma Licensing AG, 3401 Burgdorf (CH)
(72) Erfinder: Hirschel, Jürg, 3007 Bern (CH)
(74) Vertreter: Schwabe - Sandmair - Marx

(56) Entgegenhaltungen:
- WO-A1-2007/017052
- WO-A1-2009/105910
- WO-A1-2010/105376
- WO-A1-2010/139691

## Beschreibung

Die Erfindung betrifft eine Antriebs- und Dosiervorrichtung für eine Injektionsvorrichtung, die einen Mechanismus zur Verhinderung der Einstellung einer Dosis, welche die Menge des Produkts in einem Produktbehälter übersteigt, verhindert. Die Antriebs- und Dosiervorrichtung dient zur Ausschüttung eines flüssigen Produkts, insbesondere eines Medikaments. Vorzugsweise kann es sich bei dem Medikament um Insulin oder allgemein um ein Medikament zur Behandlung von Diabetes handeln.

Aus dem Stand der Technik sind Injektionsvorrichtungen, die auch als Injektionspens bezeichnet werden, bekannt. Mit solchen Injektionsvorrichtungen lassen sich individuelle, zu verabreichende Produktdosen einstellen und anschließend ausschütten. Dieser Vorgang kann mehrfach wiederholt werden. Da die Menge an Medikament in dem Produktbehälter begrenzt ist, nämlich oftmals 300 Internationale Einheiten (IU) beträgt, kann es vorkommen, dass eine Dosis eingestellt wird, die größer ist als die aus dem Produktbehälter ausschüttbare Menge, insbesondere Nennfüllmenge, die auch als in dem Produktbehälter enthaltene Menge bezeichnet werden kann. Dies bewirkt, dass z. B. 35 IU eingestellt werden, wobei mit der anschließenden Ausschüttung nur noch 20 IU ausgeschüttet werden können. Die Differenz von 15 IU fehlt dann bei der Therapie. Dies kann von dem Benutzer der Vorrichtung durchaus übersehen werden, so dass es zu gefährlichen Fehldosierungen kommen kann.

Im Stand der Technik werden daher Mechanismen vorgeschlagen, welche verhindern, dass eine Dosis eingestellt werden kann, die größer ist als die in dem Produktbehälter enthaltene Produktmenge. Die WO 01/19434 A1 schlägt z. B. in deren Figur 3 einen solchen Mechanismus vor, der eine Antriebshülse aufweist, die eine Kolbenstange umgibt und an ihrer Außenseite ein Gewinde aufweist, welches mit einem Innengewinde einer Stoppmutter in einem Eingriff ist. Die Stoppmutter weist eine Ausnehmung auf, die mit einer die Stoppmutter umgebenden Hülse in einem drehfesten und axial verschiebbaren Eingriff ist. Wird die äußere Hülse relativ zu der Antriebshülse verdreht, wie dies bei der Dosiseinstellung geschieht, wird die Stoppmutter mitgedreht und schraubt sich hierdurch an dem Gewinde der Antriebsmutter entlang zu einer Stoppposition hin. Bei der Ausschüttung der Dosis steht die Stoppmutter in Bezug auf die Antriebshülse und die äußere Hülse still, so dass ein Zählwerk gebildet wird, welches die eingestellten und ausgeschütteten Dosen zählt. Bei dem Versuch, eine Dosis einzustellen, welche die Produktmenge in dem Produktbehälter übersteigt, schlägt die Stoppmutter an einem Anschlag der Antriebshülse an und blockiert eine weitere Dosiserhöhung, so dass die Einstellung einer Dosis, welche die in dem Produktbehälter enthaltene Menge übersteigt, verhindert wird.

Eine alternative Vorrichtung mit dem gleichen Effekt wird beispielsweise in der WO 2007/017052 gezeigt. Dort wird u.a. eine Stoppmutter beschrieben, welche in einem Gewindeeingriff mit einem Außengewinde einer Kolbenstange ist. Die Kolbenstange ist für die Ausschüttung der eingestellten Dosis relativ zu dem Gehäuse drehbar und in einem Gewindeeingriff mit dem Gehäuse, so dass eine Drehung der Kolbenstange bewirkt, dass diese in Ausschüttrichtung geschraubt wird. Beim Einstellen einer Dosis, welche die in dem Produktbehälter enthaltene Produktmenge übersteigen würde, schlägt die Stoppmutter an einem an dem proximalen Ende der Kolbenstange gebildeten Anschlag an, wodurch der Mechanismus blockiert und die Einstellung einer Dosis, welche die in dem Produktbehälter enthaltene Produktmenge übersteigt, verhindert. Aus WO 2010/105376 A1 und WO 2009/105910 A1 sind Injektionsvorrichtungen mit Begrenzung einer einstellbaren Abgabedosis bekannt.

Es ist eine Aufgabe der Erfindung, eine Antriebs- und Dosiervorrichtung für eine Injektionsvorrichtung anzugeben, die eine Alternative zu den oben genannten Mechanismen bietet.

Die Aufgabe wird mit der Antriebs- und Dosiervorrichtung mit den Merkmalen nach Anspruch 1 gelöst. Vorteilhafte Weiterentwicklungen ergeben sich aus den abhängigen Ansprüchen, der Beschreibung und den Figuren.

Die erfindungsgemäße Antriebs- und Dosiervorrichtung weist ein Gehäuse auf, das z. B. mehrteilig oder einteilig sein kann. Das Gehäuse kann z. B. länglich ausgestaltet sein. Z. B. kann das Gehäuse hülsenförmig sein oder eine oder mehrere Hülsen, die z. B. konzentrisch angeordnet sein können, aufweisen. Das Gehäuse kann eine äußere Hülse aufweisen, die vom Verwender der Vorrichtung ergriffen werden kann. Das Gehäuse kann z. B. eine innere Hülse, die oftmals auch als Mechanikhalter bezeichnet wird, aufweisen, der dreh- und vorzugsweise auch axialfest mit der äußeren Hülse verbunden, insbesondere verrastet oder verklebt oder verschweißt ist. Das Gehäuse kann z. B. eine Zeigeeinrichtung, wie z. B. ein Fenster aufweisen, in der die aktuell eingestellte Dosis ablesbar ist. Das Fenster kann z. B. ein transparentes Material, insbesondere eine Linse, aufweisen oder ein Durchbruch durch das Gehäuse sein.

Am distalen Ende des Gehäuses kann ein z. B. hülsenförmiger Produktbehälterhalter, der auch als Produktbehälteraufnahme bezeichnet werden kann, lösbar oder unlösbar befestigt sein oder werden. In dem Produktbehälterhalter ist ein Produktbehälter, bzw. eine Karpule, aufgenommen oder aufnehmbar.

Die Vorrichtung umfasst ferner eine Kolbenstange, die ein distales Ende, ein proximales Ende und ein Gewinde, insbesondere ein Außengewinde aufweist und für eine Produktausschüttung mit Bezug auf das Gehäuse in eine Ausschüttrichtung bewegbar ist. Das Gewinde ist zwischen dem distalen und proximalen Ende angeordnet. An dem distalen Ende der Kolbenstange kann z. B. ein tellerförmiger Flansch angeordnet sein, der gegen den an der Abtriebs- und Dosiervorrichtung befestigten oder befestigbaren Produktbehälter drücken und diesen hierdurch verschieben kann. Die Kolbenstange ist bevorzugt in Bezug auf das Gehäuse in und insbesondere gegen die Ausschüttrichtung verschiebbar, und drehfest. Die Kolbenstange kann von dem Gehäuse oder einem zumindest drehfest mit dem Gehäuse verbundenen Führungsglied verdrehgesichert werden. Das Führungsglied kann in Bezug auf das Gehäuse verdrehfest sein, wobei die Kolbenstange ebenfalls verdrehfest an dem Führungsglied gelagert sein kann. Vorzugsweise ist die Kolbenstange relativ zu dem Führungsglied axial, d. h. entlang der Längsachse der Vorrichtung, verschiebbar. Die Kolbenstange kann z. B. einen unrunden Querschnitt, insbesondere eine oder mehrere, wie z.B. zwei, sich entlang der Längsachse der Kolbenstange erstreckende Nuten oder Abflachungen, aufweisen, in welchen das Führungsglied eingreift, so dass die Kolbenstange axial verschiebbar und drehfest ist. Das Führungsglied kann z. B. in Bezug auf das Gehäuse axialfest oder verschiebbar sein. Das Führungsglied kann ein Teil des Gehäuses oder ein separates Teil sein, insbesondere dann, wenn das Führungsglied entlang der Längsachse der Vorrichtung relativ zu dem Gehäuse verschiebbar ist. Wenn das Führungsglied in Bezug auf das Gehäuse dreh- und axialfest ist, kann es im Besonderen als Teil des Gehäuses erachtet werden.

Die Antriebs- und Dosiervorrichtung weist ferner ein Rotationsglied auf, welches operativ, insbesondere verdrehfest, mit einem Stoppanschlag verbunden ist, und in das Gewinde der Kolbenstange eingreift. Eine Drehung des Rotationsglieds relativ zu der Kolbenstange bewirkt, dass die Kolbenstange entlang der Längsachse der Vorrichtung relativ zu dem Gehäuse und mit ihrem distalen Ende voran in die Ausschüttrichtung bewegt wird. Das Rotationsglied kann in Bezug auf das Gehäuse axial fest oder nur beschränkt axial bewegbar sein. Durch Drehung des Rotationsglieds relativ zu dem Gehäuse wird die Kolbenstange an dem Rotationsglied in distale Richtung geschraubt, d.h. zwischen Rotationsglied und Kolbenstange findet eine Schraubbewegung statt, und an dem Gehäuse oder dem Führungsglied längsgeführt, so dass sich die Kolbenstange in Ausschüttrichtung (um einen Ausschütthub) axial verschiebt und sich dabei nicht dreht, d. h. drehfest in Bezug auf das Gehäuse ist.

Ferner ist ein Dosiseinstellelement vorgesehen, welches zur Erhöhung einer aus dem Produktbehälter auszuschüttenden Dosis relativ zu dem Gehäuse und/oder der Kolbenstange in eine erste Drehrichtung drehbar ist. Das Dosiseinstellelement kann insbesondere während der Dosiseinstellung und -ausschüttung in Bezug auf das Gehäuse axialfest sein. Vorzugsweise ist das Dosiseinstellelement aber in Bezug auf das Gehäuse schraubbar, so dass das Dosiselement durch seine Drehbewegung in die erste Drehrichtung aus dem Gehäuse heraus schraubbar ist und durch seine Drehbewegung in die entgegengesetzte Drehrichtung, d. h. in die zweite Drehrichtung, in das Gehäuse zurück schraubbar ist. Vorzugsweise ist das Dosiseinstellelement an dem proximalen Ende des Gehäuses angeordnet und kann aus dem proximalen Ende des Gehäuses heraus geschraubt und in das proximale Ende des Gehäuses hinein geschraubt werden. Vorzugsweise kann das Dosiseinstellelement zur Verringerung der auszuschüttenden Dosis und/oder zur Produktausschüttung in das Gehäuse hinein geschraubt werden oder zumindest in die zweite Drehrichtung gedreht werden. Durch Drehung des Dosiseinstellelements in die zweite Drehrichtung kann eine Dosisverringerung oder Dosiskorrektur durchgeführt werden. Das Dosiseinstellelement ist vorzugsweise vom Benutzer der Vorrichtung greifbar und relativ zu dem Gehäuse verdrehbar. Das Dosiseinstellelement kann auch als Dosierknopf bezeichnet werden.

Die Antriebs- und Dosiervorrichtung umfasst ferner ein Begrenzungselement, welches ein Gewinde aufweist, welches in ein weiteres Gewinde der Kolbenstange oder das Gewinde, in welches das Rotationsglied eingreift, eingreift. Das Dosiseistellelement ist so mit dem Begrenzungselement gekoppelt, dass eine Drehung des Dosiseinstellelements relativ zu der Kolbenstange und/oder zu dem Gehäuse in die erste Drehrichtung eine Drehung des Begrenzungselements relativ zu der Kolbenstange bewirkt, vorzugsweise in die gleiche Drehrichtung wie das Dosiseinstellelement. Durch die Drehung des Dosiseinstellelements und/oder des Begrenzungselements, insbesondere in die erste Drehrichtung, kann das Begrenzungselement zu dem distalen Ende der Kolbenstange hin geschraubt werden. Das Begrenzungselement weist einen Gegenanschlag auf, der durch die Drehung des Dosiseinstellelements in die erste Drehrichtung zu dem Stoppanschlag oder zu einer Stoppposition hin bewegt wird. Der entlang einer helixförmigen Kurve, die dem Gewinde, in welches das Begrenzungselement eingreift, entspricht, gemessene Abstand zwischen dem Stoppanschlag und dem Gegenanschlag ist proportional zu der (ausschüttbaren) Menge, insbesondere entweder Nennfüllmenge oder Ausschüttmenge, des Produkts in dem Produktbehälter. Dieser Abstand wird durch Drehen des Dosiseinstellelements in die erste Drehrichtung verringert. Wenn der Gegenanschlag an dem Stoppanschlag anschlägt blockiert das Begrenzungselement die Einstellung einer Dosis, z.B. welche die Menge des Produkts in dem Produktbehälter übersteigt, oder - anders ausgedrückt - die Drehung des Dosiseinstellelements in die erste Drehrichtung. Dadurch kann die Einstellung einer Dosis verhindert werden, welche die Menge des Produkts in dem Produktbehälter, d.h. die Nennfüllmenge, übersteigt, auch wenn mit der Antriebs- und Dosiervorrichtung grundsätzlich eine höhere Dosis einstellbar wäre bzw. in der Zeigeeinrichtung ein Wert der Dosisskala erscheint, der geringer ist als der Maximalwert der Dosisskala ist. Alternativ kann dadurch die Einstellung einer Dosis verhindert werden, welche bewirken würde, dass in dem Produktbehälter weniger als die Restmenge der Nennfüllmenge verbleibt, oder dass deren Ausschüttung auch die Ausschüttung zumindest einer Teilmenge der Restmenge bewirken würde; insbesondere auch wenn mit der Antriebs- und Dosiervorrichtung grundsätzlich eine höhere Dosis einstellbar wäre bzw. in der Zeigeeinrichtung ein Wert der Dosisskala erscheint, der geringer ist als der Maximalwert der Dosisskala. Es wird insbesondere auf den unten beschriebenen weiteren Aspekt Bezug genommen. Die eingestellte Dosis oder die in dem Produktbehälter enthaltene ausschüttbare Dosis kann über die Zeigeeinrichtung an einer Dosisanzeigetrommel abgelesen werden. Hierdurch wird dem Benutzer der Vorrichtung zuverlässig angezeigt, welche Dosis er sich mit der Vorrichtung gesichert verabreichen kann.

Dadurch, dass das Begrenzungselement an dem insbesondere drehfest mit dem Rotationsglied verbundenen Stoppanschlag anschlägt, wird ein äußerst stabiler Mechanismus erzielt, da sich die Teile Rotationsglied, Kolbenstange und Begrenzungselement in Bezug zueinander sperren, wenn der Gegenanschlag an dem Stoppanschlag anschlägt, d. h. das Begrenzungselement in seiner Stoppposition ist.

Besonders bevorzugt bildet das Rotationsglied den Stoppanschlag. Das Rotationsglied kann ein- oder mehrteilig sein. Ist das Rotationsglied mehrteilig, ist es bevorzugt, dass die das Rotationsglied bildenden Teile fest miteinander verbunden sind und/oder sich wie ein einziges Teil verhalten. Der Vorteil an einem mehrteiligen Rotationsglied ist, dass ein Teil aus einem ersten Kunststoff und das andere Teil aus einem zweiten Kunststoff, welcher anders ist als der erste Kunststoff, d. h. andere Eigenschaften aufweist, gebildet sein kann. Z. B. kann das Teil, welches im Gewindeeingriff mit der Kolbenstange ist, optimierte Reibeigenschaften, d. h. mit der Kolbenstange eine Reibpaarung mit geringem Reibkoeffizienten bilden. Das andere Teil, das z. B. den Stoppanschlag bildet, kann hinsichtlich seiner Festigkeit optimiert sein, d. h. z. B. aus einem faserverstärktem Kunststoff gebildet sein. Das letztgenannte Teil kann z. B. eine oder mehrere Kupplungsstrukturen aufweisen, wie z. B. Verzahnungen. Insbesondere bei Kupplungsstrukturen ist es von Vorteil, ein Augenmerk auf eine erhöhte Festigkeit zu legen.

In bevorzugten Weiterbildungen der Erfindung kann das Dosiseinstellelement zur Verringerung einer oder der eingestellten Dosis in die zweite, der ersten Drehrichtung entgegengesetzte Drehrichtung drehbar sein. Die Drehung des Dosiseinstellelements relativ zu der Kolbenstange in die zweite Drehrichtung kann eine Drehung des Begrenzungselements relativ zu der Kolbenstange, vorzugsweise ebenfalls in die zweite Drehrichtung, bewirken. Durch diese Drehung kann das Begrenzungselement zu dem proximalen Ende der Kolbenstange hin geschraubt werden. Dabei wird das Begrenzungselement vorzugsweise auch entlang der Längsachse des Gehäuses bewegt. Insbesondere wird durch die Drehung des Dosiseinstellelements und/oder des Begrenzungselements relativ zu der Kolbenstange während der Dosiseinstellung bewirkt, dass der Gegenanschlag von dem Stoppanschlag weg bewegt wird. D. h., dass der Abstand zwischen dem Gegenanschlag und dem Stoppanschlag zunimmt.

Die Antriebs- und Dosiervorrichtung umfasst vorteilhaft ein Betätigungselement, welches für die Ausschüttung der eingestellten Dosis von dem Benutzer der Vorrichtung betätigt, vorzugsweise gedrückt wird. Das Betätigungselement ist vorzugsweise am proximalen Ende der Antriebs- und Dosiervorrichtung angeordnet. Insbesondere kann das Betätigungselement bei der Betätigung relativ zu dem Dosiseinstellelement entlang der Längsachse und/oder in distale Richtung bewegt werden. Das Betätigungselement kann von dem Dosiseinstellelement aufgenommen sein. Beispielsweise kann das Betätigungselement das proximale Ende der Antriebs- und Dosiervorrichtung bilden. Das Betätigungselement kann insbesondere als Betätigungsknopf ausgestaltet sein. Das Betätigungselement ist zweckmäßigerweise so angeordnet, dass der Benutzer der Vorrichtung das Betätigungselement mit dem Daumen der Hand, welche das Gehäuse der Antriebs- und Dosiervorrichtung umgreift, betätigen, insbesondere drücken kann. Das Betätigungselement kann aus einer unbetätigten Position relativ zu dem Gehäuse und/oder dem Dosiseinstellelement in distale Richtung in eine betätigte Position gedrückt werden. Vorzugsweise ist eine Feder vorgesehen, die beim Betätigen des Betätigungselements gespannt wird. Wird das Betätigungselement losgelassen, kann es aus der betätigten Position in die unbetätigte Position verschoben werden, insbesondere durch die vorgespannte Feder.

In Ausführungsformen, bei denen das Dosiseinstellelement für die Dosiserhöhung aus dem proximalen Ende des Gehäuses heraus geschraubt wird, ist es bevorzugt, dass das Drücken des Betätigungselements zunächst eine Bewegung des Betätigungselements relativ zu dem Dosiseinstellelement in distale Richtung bewirkt (Betätigungshub des Betätigungselements), wobei ein weiteres Drücken des Betätigungselements ein Zurückschrauben des Dosiseinstellelements in das Gehäuse bewirkt (Antriebshub des Dosiseinstellelements bzw. des Betätigungselements).

Bevorzugt bewirkt das Drücken des Betätigungselements durch den Benutzer um den Antriebshub, dass das Rotationsglied relativ zu dem Gehäuse und der Kolbenstange, insbesondere in die zweite Drehrichtung, gedreht wird, wodurch sich die Kolbenstange relativ zu dem Gehäuse in die Ausschüttrichtung bewegt. Gleichzeitig wird das Begrenzungselement in Richtung zu dem proximalen Ende der Kolbenstange hin geschraubt, wobei der Abstand zwischen dem Stoppanschlag und dem Gegenanschlag idealerweise konstant bleibt. Durch das Zurückschrauben des Dosiseinstellelements in das Gehäuse wird das Rotationsglied relativ zu dem Gehäuse und der Kolbenstange, insbesondere in die zweite Drehrichtung, gedreht, wodurch sich die genannten Effekte ergeben. Das Begrenzungselement steht bei dieser Bewegung, d. h. während des Ausschüttens relativ zu dem Gehäuse axialfest und dreht sich in die zweite Drehrichtung. Hierdurch wird erreicht, dass der Abstand zwischen dem Stoppanschlag und dem Gegenanschlag konstant bleibt, sich das Begrenzungselement aber in Bezug auf die Kolbenstange in proximale Richtung schraubt, da die Kolbenstange während der Produktausschüttung keine Drehbewegung, sondern eine reine Axialbewegung ausführt.

Insbesondere ist es bevorzugt, dass zwischen dem Gehäuse und dem Rotationsglied eine unidirektionale Kupplung, insbesondere eine Ratsche, angeordnet ist, welche die Drehung des Rotationsglieds in eine Drehrichtung, welche die Bewegung der Kolbenstange in die Ausschüttrichtung bewirkt, vorzugsweise in die zweite Drehrichtung, z.B. unter Aufwendung eines geringen Drehmoments, welches ein nachgiebiges oder elastisches Mittel (Feder oder Sperrklinke) der unidirektionalen Kupplung elastisch verformt, zulässt, und in die entgegengesetzte Drehrichtung, insbesondere in die erste Drehrichtung, nicht zulässt. Vorteilhaft lässt sich hierdurch erreichen, dass bei dem Versuch das Dosiseinstellelement in die erste Drehrichtung zu drehen, wenn der Gegenanschlag des Betätigungselements an dem Anschlag des Rotationsglieds anschlägt, d. h. das Begrenzungselement in seiner Stoppposition ist, die Drehung des Dosiseinstellelements in die erste Drehrichtung verhindert wird. Vorteilhaft wird das auf das Dosiseinstellelement in die erste Drehrichtung ausgeübte Drehmoment über das Begrenzungselement in das Rotationsglied und von dem Rotationsglied über die unidirektionale Kupplung in das Gehäuse geleitet, wodurch die Drehung des Dosiseinstellelements in die erste Drehrichtung blockiert ist, wenn sich das Begrenzungselement in seiner Stoppposition befindet.

Alternativ zu der unidirektionalen Kupplung kann zwischen dem Gehäuse und dem Rotationsglied eine bidirektional wirkende Kupplung vorgesehen sein, die im eingekuppelten Zustand die Drehung des Rotationsglieds relativ zu dem Gehäuse in die erste Drehrichtung und in die zweite Drehrichtung sperrt bzw. verhindert, insbesondere wenn das Betätigungselement unbetätigt oder in seiner unbetätigten Position ist, und im ausgekuppelten Zustand, insbesondere wenn das Betätigungselement betätigt oder in seiner Betätigungsposition ist, die Drehung des Rotationsglieds relativ zu dem Gehäuse erlaubt, insbesondere in beide Drehrichtung oder zumindest in die zweite Drehrichtung erlaubt. Die bidirektionale Kupplung kann durch das Betätigen, d.h. Bewegen des Betätigungselements aus seiner unbetätigten Position in die betätigte Position, ausgekuppelt werden. Hierdurch lässt sich ebenfalls erreichen, dass bei dem Versuch das Dosiseinstellelement in die erste Drehrichtung zu drehen, wenn der Gegenanschlag des Betätigungselements an dem Anschlag des Rotationsglieds anschlägt, d. h. das Begrenzungselement in seiner Stoppposition ist, die Drehung des Dosiseinstellelements in die erste Drehrichtung verhindert wird. Vorteilhaft wird das auf das Dosiseinstellelement in die erste Drehrichtung ausgeübte Drehmoment über das Begrenzungselement in das Rotationsglied und von dem Rotationsglied über die bidirektionale Kupplung in das Gehäuse geleitet, wodurch die Drehung des Dosiseinstellelements in die erste Drehrichtung blockiert ist, wenn sich das Begrenzungselement in seiner Stoppposition befindet.

Insbesondere in Ausführungen, bei denen ein verbrauchter, d. h. entleerter Produktbehälter gegen einen neuen, d. h. gefüllten Produktbehälter ausgetauscht werden kann, ist es vorteilhaft, dass die unidirektionale Kupplung zwischen einem aktivierten Schaltzustand und einem inaktivierten Schaltzustand hin und her schaltbar ist. In ihrem aktivierten Schaltzustand lässt die unidirektionale Kupplung die Drehung des Rotationsglieds in die Drehrichtung, welche die Bewegung der Kolbenstange in die Ausschüttrichtung bewirkt, insbesondere in die zweite Drehrichtung, zu und in die entgegengesetzte Drehrichtung, insbesondere die erste Drehrichtung, nicht zu. In ihrem inaktivierten Schaltzustand lässt die unidirektionale Kupplung die Drehung des Rotationsglieds in die entgegengesetzte Drehrichtung, insbesondere die erste Drehrichtung, zu. Die unidirektionale Kupplung nimmt ihren inaktivierten Schaltzustand vorzugsweise dann ein, wenn der Produktbehälterhalter von dem Gehäuse gelöst ist oder an dem Gehäuse befestigt ist, aber kein Produktbehälter in den Produktbehälterhalter eingelegt ist. Die unidirektionale Kupplung kann in ihrem aktivierten Schaltzustand sein, wenn der Produktbehälterhalter ohne Produktbehälter oder erst mit Produktbehälter an dem Gehäuse befestigt wird. Hieraus ergeben sich zwei verschiedene Ausführungsformen, nämlich eine Ausführungsform, bei der der Produktbehälterhalter einen Mechanismus aktiviert, der die unidirektionale Kupplung in ihren aktivierten Schaltzustand schaltet, oder eine Ausführungsform, bei der der Produktbehälter diesen Mechanismus aktiviert.

Insbesondere kann zwischen dem Produktbehälter oder dem Produktbehälterhalter und der unidirektionalen Kupplung ein Schaltglied angeordnet sein, welches durch das Befestigen des Produktbehälterhalters, mit oder ohne Produktbehälter, relativ zu dem Gehäuse, insbesondere in die proximale Richtung, verschiebbar ist und die unidirektionale Kupplung in ihren aktivierten Schaltzustand schaltet. Das Schaltglied kann durch Lösen des Produktbehälterhalters oder des Produktbehälters, insbesondere in die distale Richtung verschiebbar sein oder verschoben werden und die unidirektionale Kupplung in ihren inaktivierten Schaltzustand schalten. Vorzugsweise kann eine Feder vorgesehen sein, die beim Verschieben des Schaltglieds in die proximale Richtung gespannt wird und die das Schaltglied beim Entfernen des Produktbehälterhalters oder des Produktbehälters in distale Richtung verschiebt.

Das Schaltglied kann z. B. relativ zu dem Gehäuse drehfest und entlang der Längsachse der Vorrichtung verschiebbar angeordnet sein. Z. B. kann der Produktbehälterhalter mit einer Drehbewegung an dem Gehäuse befestigt werden. Der Produktbehälterhalter kann eine Getriebefläche, die als Aktivierungselement wirkt, aufweisen, welche bei der Drehbewegung des Produktbehälterhalters relativ zu dem Gehäuse oder dem Schaltglied an dem Schaltglied abgleitet und hierdurch eine Bewegung des Schaltglieds entlang der Längsachse des Gehäuses bzw. der Vorrichtung bewirkt. Der Produktbehälterhalter kann z. B. mit einem Gewinde an dem Gehäuse angeschraubt werden. Alternativ kann der Produktbehälterhalter mittels eines Bajonettverschlusses an dem Gehäuse befestigt werden. Der Vorteil eines Bajonettverschlusses ist, dass dieser in der Regel eine Befestigung des Produktbehälterhalters an dem Gehäuse durch Drehung des Produktbehälterhalters kleiner als eine Umdrehung relativ zu dem Gehäuse erlaubt, wie z. B. um 35°, 45° oder 90°, vorzugsweise kleiner/gleich 90°. Der Bajonettverschluss kann derart sein, dass zunächst eine reine Steckbewegung und anschließend eine reine Drehbewegung des Produktbehälterhalters relativ zu dem Gehäuse für die Befestigung ausgeführt werden. Alternativ kann der Bajonettverschluss so ausgestaltet sein, dass der Produktbehälterhalter bei der Befestigung mit einer gleichzeitigen, kombinierten Dreh-Axialbewegung relativ zu dem Gehäuse befestigt wird. Besonders bevorzugt ist bei allen Ausführungen, dass der Bajonettverschluss so ausgestaltet ist, dass der Produktbehälterhalter am Ende der Befestigungsbewegung eine reine Drehbewegung, d. h. ohne Bewegung des Produktbehälterhalters entlang der Längsachse des Gehäuses ausführt.

In Ausführungen, in denen der verbrauchte Produktbehälter gegen einen neuen ausgetauscht werden kann, ist es bevorzugt, dass die Kolbenstange zurückgesetzt, d. h. in das Gehäuse der Antriebs- und Dosiervorrichtung, insbesondere in proximale Richtung, zurückgeschoben werden kann. Bei der Rücksetzung wird die Kolbenstange relativ zu dem Gehäuse in proximale Richtung bewegt. Dies kann dadurch erfolgen, dass bei gelöstem Produktbehälter oder Produktbehälterhalter, insbesondere bei inaktivierter unidirektionaler Kupplung, gegen das distale Ende der Kolbenstange gedrückt wird, wie z. B. durch einen Finger des Benutzers oder durch den Kolben des neuen Produktbehälters. Hierfür ist es bevorzugt, dass die Gewindesteigung der ineinandergreifenden Gewinde des Rotationsglieds und der Kolbenstange zumindest so groß ist, dass keine Selbsthemmung auftritt, wenn die Kolbenstange bei inaktivierter unidirektionaler Kupplung gegen die Ausschüttrichtung zurückgesetzt wird. Beim Zurücksetzen oder Verschieben der Kolbenstange in die proximale Richtung führt das Rotationsglied eine Drehung in die Drehrichtung aus, die der Drehrichtung für die Produktausschüttung entgegengesetzt ist. Insbesondere führt das Rotationsglied beim Zurücksetzen eine Drehung in die erste Drehrichtung relativ zu dem Gehäuse und/oder der Kolbenstange aus. Die Kolbenstange ist insbesondere mittels des Führungsglieds, relativ zu dem Gehäuse drehfest, wenn die Kolbenstange zurückgesetzt wird.

Vorzugsweise ist das Begrenzungselement beim Zurücksetzen der Kolbenstange in Bezug auf die Kolbenstange verdrehgesichert und axial fest, so dass das Begrenzungselement zusammen mit der Kolbenstange relativ zu dem Gehäuse in proximale Richtung verschoben wird.

Während des Zurücksetzens der Kolbenstange führt das Dosiseinstellelement relativ zu dem Gehäuse keine Drehbewegung und/oder Axialbewegung aus.

In bevorzugten Ausführungen, in denen die eingestellte Dosis besonders einfach ablesbar sein soll, kann die Antriebs- und Dosiervorrichtung eine Dosisanzeigehülse aufweisen, die zumindest drehfest, vorzugsweise auch axialfest mit dem Dosiseinstellelement gekoppelt, insbesondere mittelbar oder unmittelbar gekoppelt ist. In besonders bevorzugten Ausführungen kann die Dosisanzeigehülse mit dem Dosiseinstellelement einteilig verbunden oder verschnappt, verklebt oder verschweißt sein. Insbesondere können sich das Dosiseinstellelement und die Dosisanzeigehülse wie ein gemeinsames Teil verhalten. Die Dosisanzeigehülse kann über ihren vorzugsweise äußeren Umfang eine wendelförmig angeordnete Dosisskala aufweisen. Die Dosisskala umfasst vorzugsweise eine Vielzahl von einzelnen Dosiswerten oder zumindest Symbolen, die eine bestimmte Dosis darstellen, wobei die Werte oder Symbole so entlang einer wendelförmigen Linie angeordnet sind, dass sie die wendelförmige Dosisskala ergeben. Die Dosisskala kann z. B. Werte von 0 bis 60 oder 80 oder sogar 100 IU aufweisen, wie z. B. in 1er-, 2er- oder 5er-Schritten. Das Gehäuse kann wie gesagt eine Zeigeeinrichtung, insbesondere ein Fenster aufweisen, in dem derjenige Wert oder dasjenige Symbol der Dosisskala ablesbar ist, welcher oder welches der aktuell oder tatsächlich eingestellten oder der auszuschüttenden Dosis entspricht. Die Dosisanzeigetrommel führt mittels Drehung des Dosiseinstellelements eine Schraubbewegung relativ zu dem Gehäuse aus, so dass sich die Werte der wendelförmigen Dosisskala an der Zeigeeinrichtung entlang oder durch die Zeigeeinrichtung bewegen.

Die Dosisanzeigehülse kann z. B. ein Gewinde aufweisen, welches vorzugsweise die gleiche Steigung wie die wendelförmig angeordnete Dosisskala aufweist, wobei das Gewinde z. B. in einem Gewindeeingriff mit dem Gehäuse sein kann. Das Gehäuse kann z. B. ein Innengewinde und die Dosisanzeigetrommel ein Außengewinde aufweisen. Alternativ kann die Dosisanzeigetrommel ein Innengewinde und das Gehäuse ein Außengewinde für den Gewindeeingriff aufweisen. Die Steigung des Gewindes oder des Gewindeeingriffs ist vorzugsweise so, dass keine Selbsthemmung auftritt, wenn das Betätigungselement für die Produktausschüttung betätigt bzw. um den Antriebshub in das Gehäuse gedrückt wird, so dass sich das Dosiseinstellelement zusammen mit der Dosisanzeigetrommel in das Gehäuse schraubt, wenn das Betätigungselement (um den Antriebshub) in distale Richtung gedrückt wird.

In besonders bevorzugten Ausführungen kann die Dosisanzeigetrommel zwischen einer Nulldosisposition und einer Maximaldosisposition hin und her schraubbar sein, wobei ein Nulldosisanschlag in der Nulldosisposition der Dosisanzeigetrommel die Drehung der Dosisanzeigetrommel in die Drehrichtung, insbesondere in die zweite Drehrichtung, welche eine Verringerung der Dosis bewirkt, verhindert und in die erste Drehrichtung zulässt, und ein Maximaldosisanschlag in der Maximaldosisposition der Dosisanzeigetrommel die Drehung der Dosisanzeigetrommel in die Drehrichtung, insbesondere die erste Drehrichtung, welche eine Erhöhung der Dosis bewirkt, verhindert und in die zweite Drehrichtung, welche eine Verringerung der Dosis bewirkt, zulässt. Es ist bevorzugt, dass in der Zeigeeinrichtung der Maximalwert der Dosisskala ablesbar ist, wenn die Dosisanzeigetrommel in ihrer Maximaldosisposition ist und die Dosis Null der Dosisskala ablesbar ist, wenn die Dosisanzeigetrommel in ihrer Nulldosisposition ist. Die in der Maximaldosisposition eingestellte Dosis ist die Dosis, die mit der Antriebs- und Dosiervorrichtung maximal mit einer Ausschüttung ausgeschüttet werden kann, auch wenn in dem Produktbehälter mehr Menge des Produkts als die Maximaldosis enthalten ist. Der Maximaldosisanschlag und/oder der Nulldosisanschlag wirken vorzugsweise in Umfangsrichtung und können insbesondere als Verdrehanschläge ausgebildet sein. Gleiches kann sinngemäß für einen Maximaldosisgegenanschlag oder/und den Nulldosisgegenanschlag gelten.

In einer ersten Variante kann der Maximaldosisanschlag oder/und der Nulldosisanschlag z.B. an dem Gehäuse oder einem gehäusefesten Element, das insbesondere als zu dem Gehäuse gehörig erachtet werden kann, wie z.B. einem das Fenster der Zeigeeinrichtung bildenden Einsatz aus transparentem oder opakem Material, insbesondere Kunststoff, gebildet sein. Bevorzugt kann die Dosisanzeigetrommel den Maximaldosisgegenanschlag bilden, der in der Maximaldosisposition an dem Maximaldosisanschlag anschlägt. Die Dosisanzeigetrommel oder das Dosiseinstellelement kann den Nulldosisgegenanschlag bilden, der in der Nulldosisposition an dem Nulldosisanschlag anschlägt.

In einer zweiten Variante können der Maximaldosisanschlag oder/und der Nulldosisanschlag z.B. an einer Zwischenhülse, insbesondere Dosierhülse, angeordnet sein, die zwischen dem Dosiseinstellelement und dem Begrenzungselement angeordnet sein kann und verdrehfest mit dem Dosiseinstellelement verbunden ist. Bevorzugt kann die Zwischenhülse, insbesondere ein Ende eines Gewindes oder Gewindegangs der Zwischenhülse den Maximaldosisanschlag bilden. Alternativ oder zusätzlich kann die Zwischenhülse, insbesondere ein Ende eines Gewindes der Zwischenhülse, insbesondere das Ende des Gewindes oder des Gewindegangs, welches dem den Maximaldosisanschlag bildenden Ende entgegengesetzt ist, den Nulldosisanschlag bilden. Vorzugsweise ist der Maximaldosisanschlag distal des Nulldosisanschlags angeordnet, oder umgekehrt. Der Maximaldosisgegenanschlag und/oder der Nulldosisgegenanschlag können z.B. von einem Koppelglied, insbesondere einer Koppelhülse, gebildet werden, das in einem Gewindeeingriff mit der Zwischenhülse, insbesondere Dosierhülse, ist und drehfest und axial verschiebbar mit der Dosisanzeigetrommel verbunden ist, insbesondere in einem drehfesten und axial verschiebbaren Eingriff mit der Dosisanzeigetrommel ist. Das Koppelglied kann ein Innengewinde, insbesondere mindestens ein Innengewindesegment aufweisen, welches in ein Außengewinde, insbesondere in das Gewinde der Zwischenhülse, welches wenigstens eines aus Maximaldosisanschlag und Nulldosisanschlag bildet, eingreift, wobei bevorzugt ist, dass ein Ende des Gewindesegments den Nulldosisgegenanschlag und das andere Ende des gleichen oder eines anderen Gewindesegments den Maximaldosisgegenanschlag bildet.

Die erste Variante und die zweite Variante sind alternativ kombinierbar. Beispielsweise können der Nulldosisanschlag oder/und der Nulldosisgegenanschlag gemäß der ersten Variante und der Maximaldosisanschlag oder/und der Maximaldosisgegenanschlag gemäß der zweiten Variante gebildet sein. Alternativ können der Maximaldosisanschlag oder/und der Maximaldosisgegenanschlag gemäß der ersten Variante und der Nulldosisanschlag oder/und der Nulldosisgegenanschlag gemäß der zweiten Variante gebildet sein.

Der Gegenanschlag des Begrenzungselements schlägt an dem Stoppanschlag an und das Begrenzungselement verhindert die Drehung der Dosisanzeigetrommel in die Drehrichtung, welche eine Erhöhung der Dosis bewirkt, wenn die Dosisanzeigetrommel nicht in ihrer Maximaldosisposition ist und/oder wenn die Menge des Produkts in dem Produktbehälter geringer als die Dosis in der Maximaldosisposition der Dosisanzeigetrommel ist.

Mit anderen Worten lässt sich an der Antriebs- und Dosiervorrichtung so oft die Maximaldosis einstellen und ausschütten, wie diese Maximaldosis auch tatsächlich an Menge des Produkts in dem Produktbehälter enthalten ist. Ist die Menge des Produkts in dem Produktbehälter geringer als die Maximaldosis, schlagen Stoppanschlag und Gegenanschlag aneinander, so dass eine Erhöhung der Dosis verhindert wird.

In bevorzugten Ausführungen ist das Begrenzungselement zumindest während der Dosiseinstellung und des Ausschüttens der eingestellten Dosis drehfest mit der Dosisanzeigetrommel und/oder dem Dosiseinstellelement gekoppelt. Die Kopplung kann insbesondere in allen Betriebszuständen permanent sein. Z. B. kann das Begrenzungselement mittelbar, wie z. B. über eine Zwischenhülse mit der Dosisanzeigetrommel oder/und dem Dosiseinstellelement gekoppelt sein. Alternativ kann das Begrenzungselement unmittelbar mit dem Dosiseinstellelement oder der Dosisanzeigetrommel gekoppelt sein oder in eines der genannten eingreifen. Vorzugsweise sind die Dosisanzeigetrommel und/oder das Dosiseinstellelement in Bezug auf das Begrenzungselement entlang der Längsachse der Antriebs- und Dosiervorrichtung bewegbar.

In bevorzugten Ausführungen kann, insbesondere kinematisch, zwischen dem Dosiseinstellelement und dem Begrenzungselement mindestens eine Zwischenhülse, wie z. B. eine Antriebshülse und/oder Dosierhülse, oder eine erste und z. B. zweite Koppelhülse, angeordnet sein, in die das Begrenzungselement verdrehfest und axial verschiebbar eingreift. Die mindestens eine Zwischenhülse ist vorzugsweise verdrehfest und optional axial verschiebbar mit dem Dosiseinstellelement gekoppelt. Dies bewirkt, dass die Drehung des Dosiseinstellelements auf das Begrenzungselement übertragen wird, das in die gleiche Drehrichtung wie das Dosiseinstellelement gedreht wird.

Das Begrenzungselement ist so mit dem Dosiseinstellelement gekoppelt, dass das Begrenzungselement während der Dosiseinstellung, d. h. bei der Drehung in die erste Drehrichtung und/oder bei der Drehung in die zweite Drehrichtung, und der Verabreichung der eingestellten Dosis an der Kolbenstange entlang geschraubt wird. Insbesondere wird das Begrenzungselement bei der Dosiserhöhung in Richtung zu dem distalen Ende der Kolbenstange hin und bei der Dosisverringerung und bei der Dosisausschüttung in Richtung zu dem proximalen Ende der Kolbenstange hin geschraubt. Durch die erfindungsgemäße Anordnung wird ferner bewirkt, dass das Begrenzungselement in der Nulldosisposition unmittelbar nach einer Ausschüttung einer eingestellten Dosis in Bezug auf die Kolbenstange die gleiche Position einnimmt wie in der Nulldosisposition unmittelbar vor der Einstellung der Dosis für diese Ausschüttung. Mit anderen Worten nimmt das Begrenzungselement in der Nulldosisposition der Dosisanzeigehülse unabhängig vom Füllgrad des Produktbehälters oder vom Ausschütthub der Kolbenstange in Bezug auf die Kolbenstange immer die gleiche Position ein. Durch eine solche Kinematik lässt sich das Begrenzungselement platzsparend in die Antriebs- und Dosiervorrichtung integrieren.

Besonders bevorzugt weist die Antriebs- und Dosiervorrichtung eine Ausschüttkupplung auf, die mittels Betätigen, insbesondere Drücken, des Betätigungsglieds um den Betätigungshub durch den Benutzer der Vorrichtung geschlossen und durch Loslassen des Betätigungsglieds geöffnet wird oder werden kann. Insbesondere kann die Feder, die beim Betätigen des Betätigungsglieds und beim Schließen der Ausschüttkupplung gespannt wird, die Ausschüttkupplung öffnen und das Betätigungsglied in seine Ausgangsposition zurücksetzen.

Das Rotationsglied ist verdrehfest mit der Dosisanzeigetrommel und/oder dem Dosiseinstellelement gekoppelt, wenn die Ausschüttkupplung geschlossen und insbesondere das Betätigungsglied betätigt ist. Die Dosisanzeigetrommel und/oder das Dosiseinstellelement sind relativ zu dem Rotationsglied drehbar, wenn die Ausschüttkupplung geöffnet ist, d. h. das Betätigungsglied unbetätigt oder losgelassen ist, d. h. wenn sich das Betätigungsglied in seiner unbetätigten Position befindet.

Für die Dosiseinstellung ist das Betätigungsglied unbetätigt, wobei es für die Produktausschüttung betätigt ist. D. h., dass bei der Produktausschüttung die Ausschüttkupplung geschlossen ist, wobei sie während der Dosiseinstellung geöffnet ist. Die Ausschüttkupplung kann z. B. eine erste Kupplungsstruktur und eine zweite Kupplungsstruktur aufweisen, die bei geschlossener Ausschüttkupplung formschlüssig ineinander greifen und bei geöffneter Ausschüttkupplung außer Eingriff sind. Vorzugsweise können die Kupplungsstrukturen mit Bewegungen entlang der Längsachse der Antriebs- und Dosiervorrichtung miteinander in den und aus dem Eingriff gebracht werden. Sofern die Antriebs- und Dosiervorrichtung mindestens eine Zwischenhülse aufweist, kann eine Kupplungsstruktur permanent verdrehgesichert mit der Zwischenhülse, wie z. B. an einer Antriebshülse, verbunden sein, wobei die andere Kupplungsstruktur z. B. an dem Rotationsglied gebildet sein kann. Die Kupplungsstruktur, die mit der Kupplungsstruktur des Rotationsglieds in einen Eingriff gebracht wird, ist vorzugsweise permanent verdrehgesichert in Bezug auf das Begrenzungselement. Dadurch wird bewirkt, dass die Kupplungsstruktur die Drehbewegung des Begrenzungselements während der Dosiserhöhung, der Dosisverringerung und der Dosisausschüttung mitmacht.

Ein weiterer Aspekt betrifft die Begrenzung der ausschüttbaren Menge aus einem Produktbehälter. Dieser Aspekt kann nicht nur bei den hierin beschriebenen Antriebs- und Dosiervorrichtungen, sondern auch bei den z.B. aus dem Stand der Technik bekannten Mechanismen zur Verhinderung der Einstellung einer Dosis Anwendung finden, wie sie beispielsweise in der eingangs zitierten Patentliteratur beschrieben werden. Die Anmelderin behält sich vor, diesen Aspekt allgemein mit einer separaten Anmeldung oder mit zumindest hierauf gerichteten Ansprüchen weiterzuverfolgen.

Der in diesem Zusammenhang beschriebenen Lösung, wie sie insbesondere in den Ansprüchen 16 und 17 beschrieben wird, liegt die Aufgabe zu Grunde, die Therapiemöglichkeiten, welche die Injektionsvorrichtungen, welche die Einstellung einer Dosis in bestimmten Situationen verhindern, zu erweitern und hierfür eine einfache und kostengünstige Lösung bereitzustellen.

Im Stand der Technik wird vorgeschlagen, die Einstellung einer Dosis zu verhindern, welche die in dem Produktbehälter enthaltene Menge des Produkts übersteigt. Hierdurch wird verhindert, dass es zu Fehlanwendungen bzw. -dosierungen kommt, wenn der Anwender eine Dosis einstellen möchte, welche mit der Injektionsvorrichtung nicht mehr ausgeschüttet werden kann, weil die in dem Produktbehälter enthaltene Menge nicht mehr ausreicht.

Üblicherweise werden als Produktbehälter Karpulen verwendet, die oftmals eine Nennfüllmenge von 300 internationalen Einheiten (IU) haben. Es sei darauf hingewiesen, dass die Nennfüllmenge in der Regel geringer als die Gesamtfüllmenge des Produktbehälters ist. Die Nennfüllmenge kann durch Verschieben des Kolbens in dem Produktbehälter ausgeschüttet werden. Die Differenz zwischen Gesamtfüllmenge und Nennfüllmenge kann als Totmenge bezeichnet werden, die z.B. aufgrund der Geometrie des Kolbens und des Produktbehältergehäuses nicht ausgeschüttet werden kann. Die im Stand der Technik vorgeschlagenen Produktbehälter beziehen sich mit ihrer Blockierung der letzten Dosis auf die Nennfüllmenge.

Die Erfindung dieses Aspekts geht davon aus, dass aus einem Produktbehälter der Injektionsvorrichtung, insbesondere Karpule, nur eine erste Teilmenge, die als Ausschüttmenge bezeichnet wird, der Nennfüllmenge des in dem Produktbehälter - bei Auslieferung - enthaltenen Produkts ausschüttbar ist. Eine zweite Teilmenge, die als Restmenge bezeichnet wird und ≥ 1 IU ist, verbleibt in dem Produktbehälter. Die Restmenge könnte zwar durch Verschieben der Kolbenstange ausgeschüttet werden; aber eine solche Verschiebung wird von der Injektionsvorrichtung blockiert. Mit der Injektionsvorrichtung ist nur die Ausschüttmenge der Nennfüllmenge ausschüttbar und die Restmenge der Nennfüllmenge ist nicht ausschüttbar. Die Gesamtfüllmenge des Produktbehälters setzt sich zusammen aus der Totmenge und der Nennfüllmenge. Die Nennfüllmenge setzt sich zusammen aus der Ausschüttmenge und der Restmenge.

Die Injektionsvorrichtung weist ferner ein längliches und vorzugsweise hülsenförmiges Gehäuse und ein Dosiseinstellelement, das für eine Erhöhung der auszuschüttenden Dosis relativ zu dem Gehäuse in eine erste Drehrichtung drehbar ist und vorzugsweise für eine Verringerung der auszuschüttenden Dosis relativ zu dem Gehäuse in eine der ersten Drehrichtung entgegengesetzte, zweite Drehrichtung drehbar ist, auf. Für die Ausgestaltung des Gehäuses, des Dosiseinstellelements und der Dosisanzeigetrommel wird unabhängig von der konkreten Ausgestaltung und Integration des Begrenzungselements auf die hierin beschriebenen Ausführungen Bezug genommen. Gleiches gilt für das Betätigungselement.

Ein mit dem Dosiseinstellelement, vorzugsweise drehfest, gekoppeltes Begrenzungselement führt während einer Drehung des Dosiseinstellelements in die erste Drehrichtung, d.h. bei der Erhöhung der auszuschüttenden Dosis eine Bewegung zu dem Stoppanschlag hin aus. Hierdurch wird ein, insbesondere sich entlang einer helixförmigen Kurve erstreckender, Abstand, der insbesondere direkt proportional zu der Ausschüttmenge ist, zwischen dem Stoppanschlag und dem Begrenzungselement verringert.

Das mit dem Dosiseinstellelement gekoppelte Begrenzungselement führt während einer Drehung des Dosiseinstellelements in die zweite Drehrichtung, d.h. bei der Verringerung der auszuschüttenden Dosis eine Bewegung von dem Stoppanschlag weg aus. Hierdurch wird der, insbesondere sich entlang der helixförmigen Kurve erstreckende, Abstand, der insbesondere direkt proportional zu der Ausschüttmenge ist, zwischen dem Stoppanschlag und dem Begrenzungselement erhöht.

Vorzugsweise greift das Begrenzungselement in ein Gewinde ein, an dem es bei der Drehung des Dosiseinstellelements entlangschraubbar ist. Das Gewinde gibt bevorzugt die helixförmige Kurve vor.

Das Begrenzungselement führt während der Ausschüttung der eingestellten Dosis in Bezug auf den Stoppanschlag keine Bewegung aus, d.h. das Begrenzungselement steht in Bezug auf den Stoppanschlag still, wobei es z.B. in Bezug auf das Gehäuse währenddessen durchaus eine Bewegung ausführen kann.

Die Injektionsvorrichtung kann bevorzugt eine Ausschüttkupplung aufweisen, die durch Betätigen, insbesondere Drücken, eines Betätigungselements, insbesondere eines Betätigungsknopfs am proximalen Ende der Injektionsvorrichtung geschaltet werden kann. Die Ausschüttkupplung koppelt den Stoppanschlag und das Begrenzungselement so, dass das Begrenzungselement und der Stoppanschlag relativ zueinander durch Drehung des Dosiseinstellelements bewegbar sind, wenn das Betätigungselement unbetätigt, d.h. in seiner unbetätigten Position ist, und dass das Begrenzungselement und der Stoppanschlag relativ zueinander unbewegbar, insbesondere verdrehfest sind, wenn das Betätigungselement betätigt, d.h. in seiner betätigten Position ist.

Wenn das Begrenzungselement an dem Stoppanschlag anschlägt, wird die Einstellung einer Dosis verhindert, deren Ausschüttung auch die Ausschüttung einer Teilmenge der Restmenge bewirken würde. Mit anderen Worten kann die Einstellung einer Dosis verhindert werden, welche bewirken würde, dass in dem Produktbehälter weniger als die Restmenge der Nennfüllmenge verbleibt. Mit der Vorrichtung kann somit nur die Ausschüttmenge und nicht die Restmenge bzw. auch keine Teilmenge davon ausgeschüttet werden.

Die Injektionsvorrichtung kann kostengünstig durch Veränderung im Zusammenbau der hierin beschriebenen und aus dem Stand der Technik bekannten Injektionsvorrichtungen hergestellt werden. Zunächst wird die Nennfüllmenge für den Produktbehälter für die Injektionsvorrichtung festgelegt. Ferner wird die Ausschüttmenge festgelegt. Die Injektionsvorrichtung wird so montiert, dass nur die Ausschüttmenge, die kleiner ist als die Nennfüllmenge ausschüttbar ist und die Restmenge der Nennfüllmenge nicht ausschüttbar ist. Dies geschieht dadurch, dass das Begrenzungselement in Bezug auf den Stoppanschlag mit einem, insbesondere sich entlang der helixförmigen Kurve erstreckten, Abstand angeordnet wird, wobei der Abstand direkt proportional zu der Ausschüttmenge ist. Bei den Vorrichtungen aus dem Stand der Technik ist der Abstand direkt proportional zur Nennfüllmenge.

Die Erfindung wurde Anhand mehrerer bevorzugter Ausführungen und Weiterbildungen beschrieben. Im Folgenden werden besonders bevorzugte Ausführungen einer Antriebs- und Dosiervorrichtung anhand von Figuren beschrieben. Die dabei offenbarten Merkmale bilden den Gegenstand der Erfindung jeweils einzeln und in jeglicher Merkmalskombination, insbesondere auch mit einem oder mehreren der vorher genannten Merkmale, vorteilhaft weiter. Es zeigen:
- Figur 1: eine Explosionsdarstellung der Einzelteile einer Antriebs- und Dosiervorrichtung gemäß einer ersten Ausführungsform,
- Figuren 2a, 2b: die Antriebs- und Dosiervorrichtung der ersten Ausführungsform in einem Ausgangszustand mit einem vollständig gefüllten Produktbehälter, wobei Figur 2b eine in Bezug auf Figur 2a um 90° um die Längsachse gedrehte Schnittansicht ist,
- Figur 3: die Vorrichtung aus Figur 2 in einer Maximaldosisposition, wobei ein Betätigungselement unbetätigt ist,
- Figur 4: die Ansicht aus Figur 3, wobei das Betätigungselement betätigt ist,
- Figur 5: die Vorrichtung gemäß der ersten Ausführungsform, nach einer Ausschüttung bzw. in einer Nulldosisposition, nachdem die in den Figuren 3 und 4 eingestellte Dosis ausgeschüttet wurde,
- Figur 6: die Vorrichtung gemäß der ersten Ausführungsform, wobei eine Dosiserhöhung mittels eines Begrenzungselements blockiert wird,
- Figur 7: die Vorrichtung gemäß der ersten Ausführungsform, nach einer Ausschüttung bzw. in einer Nulldosisposition, nachdem die in der Figur 6 eingestellte Dosis ausgeschüttet wurde,
- Figur 8: die Vorrichtung nach der ersten Ausführungsform, wobei der Produktbehälter und ein Produktbehälterhalter von der Antriebs- und Dosiervorrichtung entfernt werden,
- Figur 9: die Antriebs- und Dosiervorrichtung nach der ersten Ausführungsform, wobei eine Kolbenstange zurückgesetzt wurde und ein neuer Produktbehälter und ein Produktbehälterhalter an der Antriebs- und Dosiervorrichtung befestigt werden,
- Figuren 10a, 10b: die Vorrichtung nach der ersten Ausführungsform, wobei ein neuer Produktbehälter eingesetzt ist, und das Betätigungsglied in der Nulldosisposition betätigt wird,
- Figur 11: eine Explosionsdarstellung der Einzelteile nach einer zweiten Ausführungsform einer Antriebs- und Dosiervorrichtung,
- Figur 12: verschiedene Schnittansichten der Antriebs- und Dosiervorrichtung der zweiten Ausführungsform in einer Nulldosisposition,
- Figur 13: die Schnittansichten aus Figur 12 in einer Maximaldosisposition,
- Figur 14: die Schnittansichten aus Figur 13, wobei die in Figur 13 eingestellte Produktdosis vollständig ausgeschüttet wurde,
- Figur 15: die Schnittansichten aus Figur 12, wobei sich das Begrenzungselement in der Stoppposition befindet,
- Figur 16: die Schnittansichten aus Figur 15, wobei die in Figur 15 eingestellte Dosis ausgeschüttet ist,
- Figur 17: verschiedene Schnittansichten der Antriebs- und Dosiervorrichtung der zweiten Ausführungsform, bei der eine unidirektionale Kupplung inaktiviert wurde, und
- Figur 18: die Schnittansichten aus Figur 17, bei der die Kolbenstange zurückgesetzt wurde.

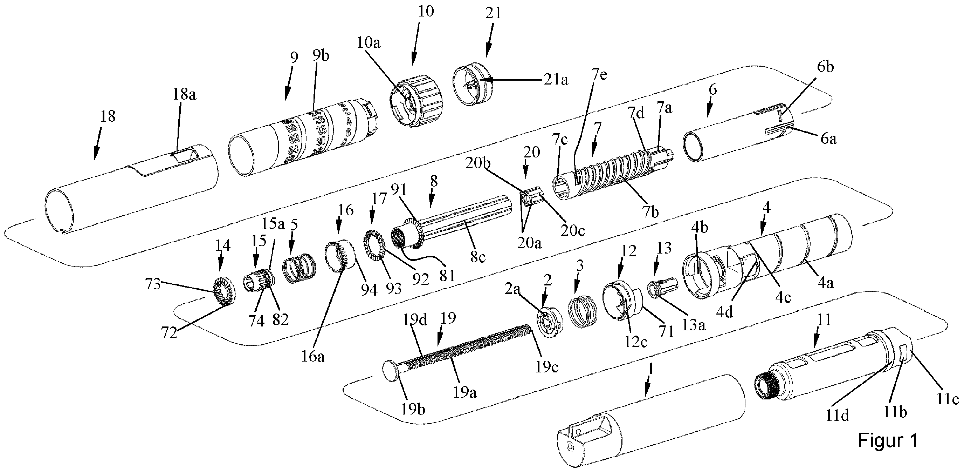

Die in den Figuren 1 bis 10 gezeigte erste Ausführungsform einer Antriebs- und Dosiervorrichtung weist ein Gehäuse 4, 18 auf, welches eine äußere Gehäusehülse 18 und eine innerhalb und insbesondere konzentrisch zu der äußeren Gehäusehülse 18 angeordnete innere Gehäusehülse 4, die auch als Mechanikhalter 4 bezeichnet wird, aufweist. Der Mechanikhalter 4 ist mit der äußeren Gehäusehülse 18 fest, d. h. dreh- und axialfest verbunden. Das Gehäuse 4, 18, nämlich der Mechanikhalter 4, weist ein Gewinde 4a auf, in welches ein am Innenumfang einer Dosisanzeigetrommel 9 gebildetes Gewinde eingreift, so dass die Dosisanzeigetrommel 9 an dem Gehäuse 4, 18 bzw. deren Längsachse L entlang schraubbar ist. Die Dosisanzeigetrommel ist fest, d. h. dreh- und axialfest mit einem Dosiseinstellelement 10 verbunden, nämlich in diesem Beispiel verrastet. Das Dosiseinstellelement 10 ist vom Benutzer der Vorrichtung greifbar und in Bezug auf das Gehäuse 4, 18 drehbar. Durch Drehen des Dosiseinstellelements 10 in eine erste Drehrichtung wird eine auszuschüttende Produktdosis erhöht und durch Drehen in die entgegengesetzte, d. h. zweite Drehrichtung verringert. Durch Drehen des Dosiseinstellelements 10 in die erste Drehrichtung wird die Dosisanzeigetrommel 9 aus dem proximalen, d. h. hinteren Ende des Gehäuses 4, 18 heraus geschraubt und durch Drehen in die zweite Drehrichtung in das Gehäuse 4, 18 hineingeschraubt.

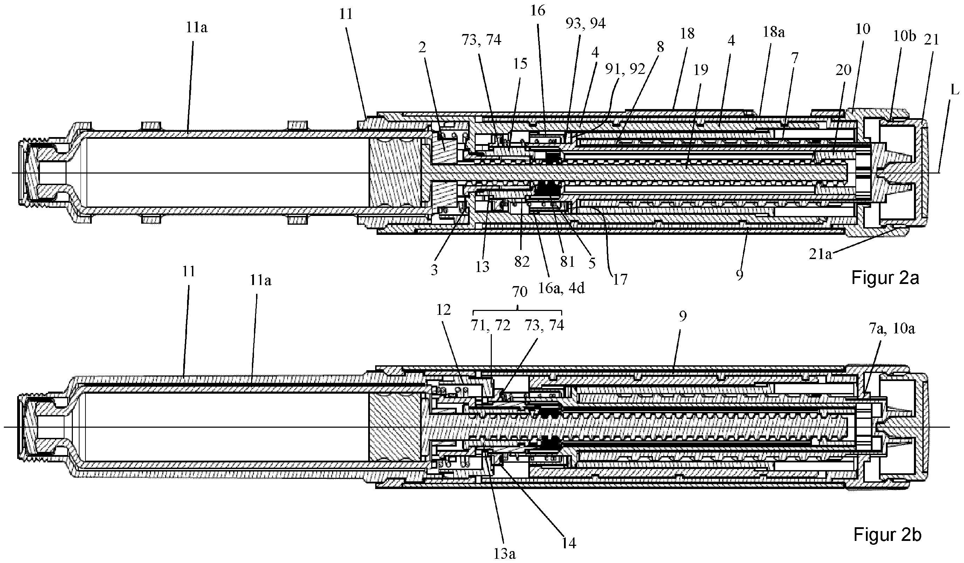

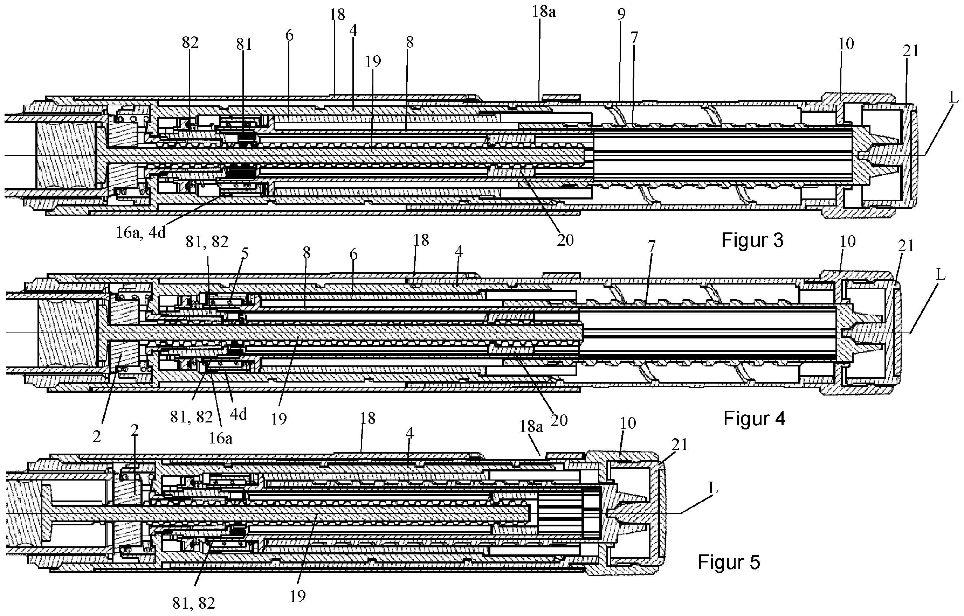

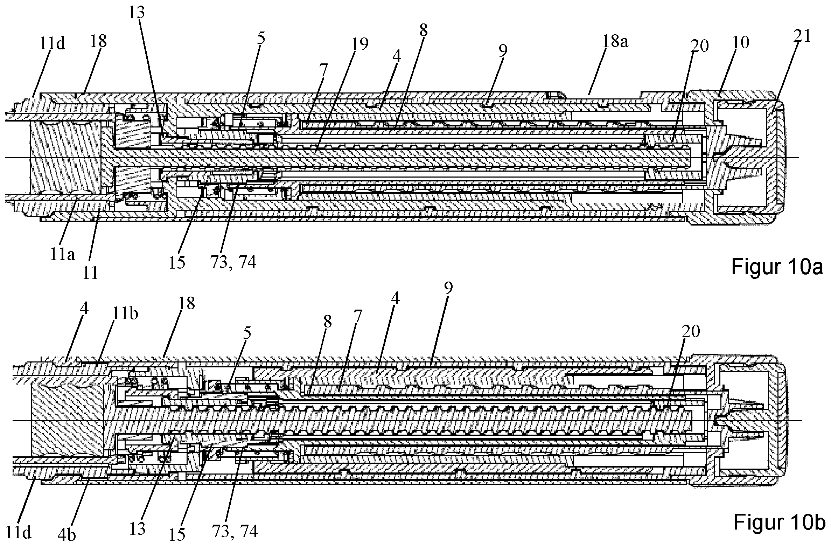

Das Gehäuse 4, 18, nämlich die äußere Gehäusehülse 18 weist eine Zeigeeinrichtung 18a auf, die in dem gezeigten Beispiel ein Durchbruch durch das Gehäuse 18 ist. In der Zeigeeinrichtung 18a kann der Benutzer der Vorrichtung die eingestellte Produktdosis ablesen, indem er durch die Zeigeeinrichtung 18a auf eine Dosisskala blickt. Die Dosisanzeigetrommel 9 weist an ihrem Außenumfang eine sich wendelförmig über ihren äußeren Umfang erstreckende Dosisskala 9b auf, die eine Vielzahl aneinander gereihte Zahlenwerte aufweist, wobei in dem gezeigten Beispiel die Zahlenwerte 0 bis 60 in Zweierschritten angegeben sind. Die Zahlenwerte repräsentieren eine Dosis in internationalen Einheiten (IU). Soll z. B. eine Dosis von 30 IU eingestellt werden, wird das Dosiseinstellelement 10 so weit in Bezug auf das Gehäuse 4, 18 gedreht, bis die Zahl 30 in der Zeigeeinrichtung 18a ablesbar ist. Am proximalen Ende der Antriebs- und Dosiervorrichtung ist ein Betätigungselement 21 in der Gestalt eines Betätigungsknopfs angeordnet, und bildet insbesondere das proximale Ende der Antriebs- und Dosiervorrichtung. Das Betätigungselement 21 ist von dem Dosiseinstellelement 10 aufgenommen, insbesondere derart aufgenommen, dass das Betätigungselement 21 einen Betätigungshub relativ zu dem Dosiseinstellelement 10 ausführen kann. Das Betätigungselement 21 weist hierfür beispielsweise eine ringförmige Nut auf, in die eine nach innen gerichtete Abragung 10b des Dosiseinstellelements eingreift. Beispielsweise wird in den Figuren 2a, 2b und 3 das Betätigungselement 21 in seiner unbetätigten Position dargestellt. In den Figuren 4 und 10a, 10b wird das Betätigungselement 21 in seiner betätigten Position dargestellt, wobei es im Vergleich zur Figur 3 einen Betätigungshub ausgeführt hat. Das Betätigungselement 21 kann aufgrund seiner Positionierung am proximalen Ende der Vorrichtung durch den Daumen der Hand des Verwenders, welche das Gehäuse 4, 18 umgreift, auf eine einfache Weise gedrückt, d. h. betätigt werden.

Zur Ausschüttung der eingestellten Produktdosis wird das Betätigungselement 21 noch weiter in distale Richtung gedrückt, so dass das Dosiseinstellelement 10 zusammen mit der Dosisanzeigetrommel 9 in das Gehäuse 4, 18 zurück geschraubt werden (vgl. Figuren 4 und 5) kann. Das Betätigungselement 21 und das Dosiseinstellelement 10 führen hierbei einen Antriebshub relativ zu dem Gehäuse 4, 18 aus.

An dem distalen Ende des Mechanikhalters 4 bzw. des Gehäuses 4, 18 ist ein hülsenförmiger Produktbehälterhalter 11 angebracht, in dem ein Produktbehälter 11a in der Gestalt einer Karpule aufgenommen ist. Die Karpule 11a weist an ihrem distalen Ende ein von einer Nadel durchstechbares Septum, sowie einen Kolben auf, der für die Produktausschüttung in Ausschüttrichtung, d. h. zu dem Septum hin verschiebbar ist, um das zwischen Septum und Kolben befindliche flüssige Produkt, wie z. B. Insulin, aus dem Produktbehälter 11a zu verdrängen. An dem Kolben stützt sich eine Kolbenstange 19 ab. Die Kolbenstange 19 weist ein vorzugsweise mehrgängiges Außengewinde 19a auf, in welches ein Innengewinde 13a des Rotationsglieds 13, 15 eingreift. Das Rotationsglied 13, 15 ist in Bezug auf das Gehäuse 4, 18 drehbar und axialfest angeordnet. Das Rotationsglied 13, 15 ist in dem gezeigten Beispiel zweiteilig und weist eine Gewindemutter 13 und eine Kupplungshülse 15 auf, die dreh- und axialfest mit einander verschnappt sind. Zwischen der Gewindemutter 13 und der Kupplungshülse 15 ist eine Ringnut gebildet, in die ein Teil des Gehäuses 4, 18, nämlich ein Teil des Mechanikhalters 4 eingreift, so dass das Rotationsglied 13, 15 relativ zu dem Gehäuse 4 drehbar aber nicht axial bewegbar ist.

Ein Führungsglied 2, welches drehfest mit dem Gehäuse 4, 18 verbunden ist und in Bezug auf das Gehäuse 4, 18 entlang der Längsachse L nicht oder zumindest begrenzt (z.B. im Bereich von einem oder mehreren Millimeter, wie z.B. kleiner 5 mm) axial bewegbar ist, führt die Kolbenstange 19 drehfest, so dass die Kolbenstange 19 relativ zu dem Gehäuse 4, 18 nicht drehbar ist. Die Kolbenstange 19 ist jedoch relativ zu dem Führungsglied 2 entlang der Längsachse L bewegbar. Die Kolbenstange 19 weist zumindest eine Führungs- und/oder Längsnut 19d auf, welche dem Außengewinde 19a überlagert ist und sich parallel zur Längsachse L erstreckt. In die Führungsnut 19d greift das Führungsglied 2 ein, so dass die Kolbenstange 19 relativ zu dem Führungsglied 2 verdrehgesichert und axial entlang der Längsachse L verschiebbar ist.

Die Antriebs- und Dosiervorrichtung weist das Rotationsglied 13, 15 auf, welches bei der Dosiseinstellung, d. h. der Dosiserhöhung oder/und -verringerung, drehfest in Bezug auf die Kolbenstange 19 und/oder das Gehäuse 4, 18 ist und für die Produktausschüttung relativ zu der Kolbenstange 19 und/oder dem Gehäuse 4, 18 um die Achse L gedreht wird, insbesondere in die zweite Drehrichtung des Dosiseinstellelements 10. Das Rotationsglied 13, 15 macht die Drehbewegung des Dosiseinstellelements 10 für die Produktausschüttung mit. Das Rotationsglied 13, 15 greift mit einem Innengewinde 13a in das Außengewinde 19a der Kolbenstange 19 ein. Das Rotationsglied 13, 15 ist axialfest und drehbar mit dem Gehäuse 4, 18, insbesondere mit dem Mechanikhalter 4 verbunden. Das Rotationsglied 13, 15 weist zwei Teile, nämlich eine Gewindemutter 13 und eine Kupplungshülse 15 auf, die miteinander dreh- und axialfest verrastet sind und sich wie ein einziges Teil verhalten. Die Gewindemutter 13 ist aus einem Kunststoff, wie z. B. Teflon, gebildet, welcher mit dem Material der Kolbenstange 19 eine Reibpaarung mit einem niedrigen Reibkoeffizienten bildet. Die Kupplungshülse 15 ist aus einem Kunststoff gebildet, der eine höhere Festigkeit aufweist als der Kunststoff der Gewindemutter 13, wie z. B. aus faserverstärktem Kunststoff. Zwischen der Gewindemutter 13 und der Kupplungshülse 15 ist ein sich über den Umfang des Rotationsglieds 13, 15 erstreckende Ringnut angeordnet, in die ein Teil des Gehäuses 4, 18 oder ein gehäusefestes Element, in diesem Beispiel der Mechanikhalter 4 eingreift. Beispielsweise begrenzen die Gewindemutter 13 und die Kupplungshülse 15 die Ringnut. Hierdurch wird eine Montage des Rotationsglieds 13, 15 erleichtert. Bevorzugt kann das Rotationsglied 13, 15 in allen Betriebszuständen der Antriebs- und Dosiervorrichtung axialfest in Bezug auf das Gehäuse 4, 18 angeordnet sein.

Eine Ausschüttkupplung 81, 82 wird mittels Drücken des Betätigungselements 21 in distale Richtung durch den Benutzer der Vorrichtung geschlossen und durch Loslassen des Betätigungselements 21 geöffnet. Die Ausschüttkupplung 81, 82 koppelt das Rotationsglied 13, 15 drehfest mit dem Dosiseinstellelement 10, wenn die Ausschüttkupplung 81, 82 geschlossen ist. Wenn die Ausschüttkupplung 81, 82 geöffnet ist, ist das Dosiseinstellelement 10 rotatorisch von dem Rotationsglied 13, 15 entkoppelt, d. h. insbesondere, dass das Dosiseinstellelement 10 relativ zu dem Rotationsglied 13, 15 verdrehbar ist.

Die Ausschüttkupplung 81, 82 weist eine erste Kupplungsstruktur 81, die auch als erste Ausschüttkupplungsstruktur bezeichnet werden kann, und eine zweite Kupplungsstruktur 82, die auch als zweite Ausschüttkupplungsstruktur bezeichnet werden kann, auf, die in einen verdrehfesten Eingriff miteinander gebracht werden, wenn die Kupplung 81, 82 geschlossen wird. Die Kupplung 81, 82 weist ferner eine Feder 5 auf, welche die erste Kupplungsstruktur 81 und die zweite Kupplungsstruktur 82 aus einem Eingriff drängt, insbesondere durch die in der Feder 5 gespeicherte Federenergie. Durch die Betätigung des Betätigungselements 21 wird die Kupplung 81, 82 geschlossen, d. h. die erste Kupplungsstruktur 81 mit der zweiten Kupplungsstruktur 82 in einen verdrehfesten Eingriff gebracht, wobei die Feder 5 gespannt wird. Die Feder 5 dient insbesondere auch zum Rücksetzen des Betätigungselements 21 in seine unbetätigte Position.

Die erste Kupplungsstruktur 81 ist in dem gezeigten Beispiel eine sich über den Umfang erstreckende Innenverzahnung, wobei die zweite Kupplungsstruktur 82 eine sich über den Umfang erstreckende Außenverzahnung ist, wobei die erste und zweite Kupplungsstruktur 81, 82 mit einer Bewegung entlang der Längsachse L in einen Eingriff gebracht werden können. Die zweite Kupplungsstruktur 82 ist insbesondere drehfest mit dem Rotationsglied 13, 15 gekoppelt. Vorzugsweise wird die zweite Kupplungsstruktur 82 von dem Rotationsglied 13, 15, insbesondere der Kupplungshülse 15 gebildet.

Die erste Kupplungsstruktur 81 kann z. B. eine Antriebshülse 8 sein, die geometrisch und/oder kinematisch zwischen dem Rotationsglied 13, 15 und dem Betätigungselement 21 angeordnet sein kann. Durch Drücken des Betätigungselements 21 wird die Antriebshülse 8 entlang der Längsachse L und relativ zu dem Gehäuse 4, 18 und/oder der Kolbenstange 19 in distale Richtung und durch Loslassen des Betätigungselements 21 mittels der in der Feder 5 gespeicherten Federenergie in proximale Richtung verschoben.

Die Antriebshülse 8 ist vorzugsweise permanent, insbesondere während der Dosiseinstellung und Dosisausschüttung drehfest mit dem Dosiseinstellelement 10 verbunden. Insbesondere ist es bevorzugt, dass die erste Kupplungsstruktur 81 permanent drehfest mit dem Dosiseinstellelement 10 verbunden ist.

Zwischen der Antriebshülse 8 und dem Betätigungselement 21 ist eine Dosierhülse 7 angeordnet, welche drehfest und axial verschiebbar in die Antriebshülse 8 eingreift. Die Dosierhülse 7 weist an ihrem inneren Umfang ein oder mehrere Führungsschlitze 7c auf, die sich parallel zur Längsachse L erstrecken und in die eine oder mehrere Führungsrippen 8c, die am äußeren Umfang der Antriebshülse 8 gebildet werden, eingreifen, um die drehfeste aber axial verschiebbare Verbindung zwischen der Dosierhülse 7 und der Antriebshülse 8 zu bilden.

Die Dosierhülse 7 weist an ihrem äußeren Umfang ein Gewinde 7b auf, welches distal von einem Maximaldosisanschlag 7e und proximal von einem Nulldosisanschlag 7d begrenzt wird. Ein Koppelglied 6 weist mindestens ein Innengewindesegment 6b auf, welches in das Gewinde 7b eingreift. Obwohl es sich um ein Innengewindesegment 6b handelt ist dieses in Figur 1 am Außenumfang des Koppelglieds 6 sichtbar, da aus herstellungstechnischen Gründen von außen Vertiefungen eingebracht sind, um Materialhäufung beim Spritzguss zu vermeiden. Das mindestens eine Innengewindesegment 6b bildet mit einem in Umfangsrichtung weisenden Ende den Maximaldosisanschlag, der in der Maximaldosisposition bei dem Versuch, das Dosiseinstellelement 10 in die erste Drehrichtung zu drehen, an den Maximaldosisanschlag 7e anschlägt, und mit dem entgegengesetzten in Umfangsrichtung weisenden Ende den Nulldosisgegenanschlag, der in der Nulldosisposition bei dem Versuch, das Dosiseinstellelement 10 in die zweite Drehrichtung zu drehen, an dem Nulldosisanschlag 7d anschlägt. Das Gewinde 7b ist mehrgängig, in diesem Beispiel viergängig. Insbesondere kann das Gewinde 7b die gleiche Steigung wie das Gewinde 4a aufweisen.

Alternativ kann der Mechanikhalter 4 bzw. das Gehäuse 4, 18 den Nulldosisanschlag 4c aufweisen, der z.B. von dem distalen Ende des Gewindes 4a gebildet sein kann. Der Nulldosisgegenanschlag kann von der Dosisanzeigetrommel 9, wie z.B. an deren Innenumfang, gebildet sein. Beispielsweise kann der Nulldosisgegenanschlag von einem Ende des in das Gewinde 4a eingreifenden Innengewindes oder Innengewindesegments der Dosisanzeigetrommel 9 gebildet werden.

Die Dosierhülse 7 ist vorzugsweise permanent drehfest und axial verschiebbar mit dem Dosiseinstellelement 10 verbunden. Hierfür weist die Dosierhülse 7 eine oder mehrere Rippen 7a auf, die sich parallel zur Längsachse L erstrecken und in eine oder mehrere Ausnehmungen 10a des Dosiseinstellelements 10 eingreifen.

Zwischen dem Mechanikhalter 4 und der Dosierhülse 7 ist das Koppelglied 6, welches als Hülse ausgestaltet ist und somit als Koppelhülse 6 bezeichnet werden kann, angeordnet. Die Koppelhülse 6 ist in einem verdrehgesicherten und axial verschiebbaren Eingriff mit dem Mechanikhalter 4. Hierfür weist die Koppelhülse 6 an ihrem äußeren Umfang eine oder mehrere Rippen 6a auf, welche in eine sich parallel zur Längsachse L erstreckende Nut des Mechanikhalters 4 eingreift. Die Koppelhülse 6 weist ein Innengewinde auf, welches in das Gewinde 7b der Dosierhülse 7 eingreift.

Das Betätigungselement 21 ist an dem proximalen Ende der Dosierhülse 7 an der Dosierhülse 7 frei drehbar befestigt.

Zwischen dem Gehäuse 4, 18 ist eine unidirektionale Kupplung 71, 72, 73, 74 angeordnet, die im Folgenden mit dem Bezugszeichen 70 allgemein beschrieben wird. Während der Dosiseinstellung und/oder -ausschüttung lässt die unidirektionale Kupplung 70 eine Drehung des Rotationsglieds 13, 15 in die erste Drehrichtung relativ zu dem Gehäuse 4, 18 und/oder der Kolbenstange 19 nicht zu und in die zweite Drehrichtung zu. Die unidirektionale Kupplung 70 kann als Ratsche ausgebildet sein. Die unidirektionale Kupplung 70 verhindert, dass bei der Dosiserhöhung das Rotationsglied 13, 15 in die erste Drehrichtung gedreht wird aufgrund von eventuell auftretenden Reibungen zwischen dem Dosiseinstellelement 10 und dem Rotationsglied 13, 15 oder aufgrund von Elastizitäten des Kolbens des Produktbehälters 11a, die versuchen, die Kolbenstange 19a in die proximale Richtung zurück zu drücken.

Sofern die Antriebs- und Dosiervorrichtung als Einwegvorrichtung ausgestaltet sein soll, die nach vollständiger Entleerung des Produktbehälters 11a entsorgt wird, kann die unidirektionale Kupplung 70 permanent wirken und nicht lösbar sein. Soll die Antriebs- und Dosiervorrichtung jedoch für eine Mehrwegverwendung vorgesehen sein, d. h. dass der Produktbehälter 11a gegen einen anderen Produktbehälter 11a ausgetauscht werden soll, ist es bevorzugt, dass die unidirektionale Kupplung 70 beim Auswechseln des Produktbehälters 11a lösbar ist, wodurch sich das Rotationsglied 13, 15 in die erste Drehrichtung relativ zu dem Gehäuse 4, 18 drehen kann, so dass die Kolbenstange 19 in ihre Ausgangsposition zurücksetzbar ist.

Die unidirektionale Kupplung 70 weist eine erste Kupplungsstruktur 71 und eine zweite Kupplungsstruktur 72 auf, wobei die erste und zweite Kupplungsstruktur 71, 72 ineinander greifen und nur in eine Drehrichtung relativ zueinander drehbar sind. Die erste Kupplungsstruktur 71 kann z. B. an dem Gehäuse 4, 18 oder zumindest verdrehgesichert mit dem Gehäuse 4, 18 verbunden sein, wie z. B. an einem Schaltglied 12 gebildet werden. Die zweite Kupplungsstruktur 72 kann z. B. von dem Rotationsglied 13, 15, insbesondere der Kupplungshülse 15 oder zumindest von einem mit dem Rotationsglied 13, 15 zumindest zeitweise drehfest verbundenen Teil, wie z. B. einem Kupplungsring 14 verbunden sein oder daran gebildet sein. Der Kupplungsring 14 ist bevorzugt drehfest und axial verschiebbar in Bezug auf das Rotationsglied, zumindest während der Dosiseinstellung und -ausschüttung. Insbesondere werden die erste und zweite Kupplungsstruktur 71, 72 von einer Feder, insbesondere der Feder 5, die sich z. B. an dem Kupplungsring 14 abstützt, in einem Eingriff gehalten. Vorzugsweise sind die erste Kupplungsstruktur 71 und die zweite Kupplungsstruktur 72 jeweils als über den Umfang verteilte sägezahnförmige Zähne ausgestaltet. Die erste Kupplungsstruktur 71 weist vorzugsweise in die proximale Richtung, wobei die zweite Kupplungsstruktur 72 vorzugsweise in distale Richtung weist. Die Zähne der ersten Kupplungsstruktur 71 und die Zähne der zweiten Kupplungsstruktur 72 weisen vorzugsweise zueinander. Die Sägezähne der Kupplungsstrukturen 71, 72 weisen eine steile Flanke und eine flache Flanke auf, wobei die flache Flanke der einen Kupplungsstruktur an der flachen Flanke der anderen Kupplungsstruktur abgleiten kann, wodurch die zweite Kupplungsstruktur 72 relativ zur ersten Kupplungsstruktur 71 in die zweite Drehrichtung drehbar ist. Bei dem Versuch die zweite Kupplungsstruktur 72 relativ zu der ersten Kupplungsstruktur 71 in die erste Drehrichtung zu verdrehen, werden die steilen Flanken der Kupplungsstrukturen 71, 72 gegeneinander gepresst, wodurch die Drehung der zweiten Kupplungsstruktur in die erste Drehrichtung gesperrt wird.

Zum Einstellen der Dosis wird das Dosiseinstellelement 10 in die erste Drehrichtung relativ zu dem Gehäuse 4, 18 gedreht, wodurch das Dosiseinstellelement 10 aus dem proximalen Ende des Gehäuses 4, 18 zusammen mit der Dosisanzeigetrommel 9 herausgeschraubt wird (Figur 3). Die eingestellte Dosis ist an der Zeigeeinrichtung 18a an der Dosisskala 9b ablesbar. Die Ausschüttkupplung 81, 82 ist während der Dosiseinstellung geöffnet. In Figur 3 wird die Vorrichtung in einem Zustand gezeigt, in dem die auszuschüttende Produktdosis eingestellt ist.

Zur Ausschüttung der in Figur 3 eingestellten Produktdosis wird das Betätigungselement 21 relativ zu dem Dosiseinstellelement 10 um einen Betätigungshub in distale Richtung gedrückt (vgl. Figuren 3 und 4), wodurch die Dosierhülse 7 ebenfalls um den Betätigungshub des Betätigungselements 21 relativ zu dem Gehäuse 4, 18 verschoben wird. Aufgrund des Gewindeeingriffs zwischen dem Dosierglied 7 und der Koppelhülse 6 wird die Koppelhülse 6 relativ zu dem Gehäuse 4, 18 in distale Richtung, nämlich um den Betätigungshub des Betätigungselements 21 verschoben. Das Koppelglied 6 stützt sich an der Antriebshülse 8 ab, wodurch die Antriebshülse 8 ebenfalls in distale Richtung verschoben wird, insbesondere um den Betätigungshub des Betätigungselements 21. Hierdurch wird die an der Antriebshülse 8 gebildete erste Kupplungsstruktur 81 in einen verdrehfesten Eingriff mit der zweiten Kupplungsstruktur 82 des Rotationsglieds 13, 15 gebracht, wodurch die Ausschüttkupplung 81, 82 geschlossen wird. Figur 4 zeigt die Vorrichtung bei betätigtem Betätigungselement 21.

Wird das Betätigungselement 21 weiter in distale Richtung gedrückt, wird das Dosiseinstellelement 10 um einen Antriebshub (vgl. Figuren 4 und 5) in das Gehäuse 4, 18 zurückgeschraubt. Hierbei zählen die Dosiswerte der Dosisskala in der Zeigeeinrichtung 18a zurück. Beim Zurückschrauben des Dosiseinstellelements 10 wird die Drehbewegung des Dosiseinstellelements 10 über die Dosierhülse 7 auf die Antriebshülse 8 und von der Antriebshülse 8 über die geschlossene Ausschüttkupplung 81, 82 auf das Rotationsglied 13, 15 übertragen, wodurch das Rotationsglied 13, 15 eine Drehbewegung relativ zu der Kolbenstange 19 und/oder zu dem Gehäuse 4, 18 in die zweite Drehrichtung, nämlich in die gleiche Drehrichtung wie das Dosiseinstellelement 10 ausführt. Durch diese Drehbewegung wird die Kolbenstange 19 in distale Richtung verschoben, wobei die Kolbenstange 19 von dem Führungsglied 2 drehfest in Bezug auf das Gehäuse 4, 18 gehalten wird. Der am distalen Ende der Kolbenstange 19 angeordnete Flansch drückt gegen den Kolben und verschiebt den Kolben des Produktbehälters 11a in Ausschüttrichtung, so dass das in dem Produktbehälter 11a enthaltene Produkt entsprechend der eingestellten Produktdosis aus dem Produktbehälter 11a verdrängt wird. Die Kolbenstange 19 führt einen Abtriebshub aus. Vorzugsweise ist der Antriebshub des Dosiseinstellelements 10 proportional zu dem und größer als der Abtriebshub der Kolbenstange 19, wodurch eine Übersetzung gebildet wird und die für die Ausschüttung auf das Betätigungselement 21 auszuübende Kraft zur Verschiebung des Kolbens abnimmt.

Dieser Vorgang kann mehrmals wiederholt werden, wobei jeweils eine individuelle Dosis einstellbar ist.

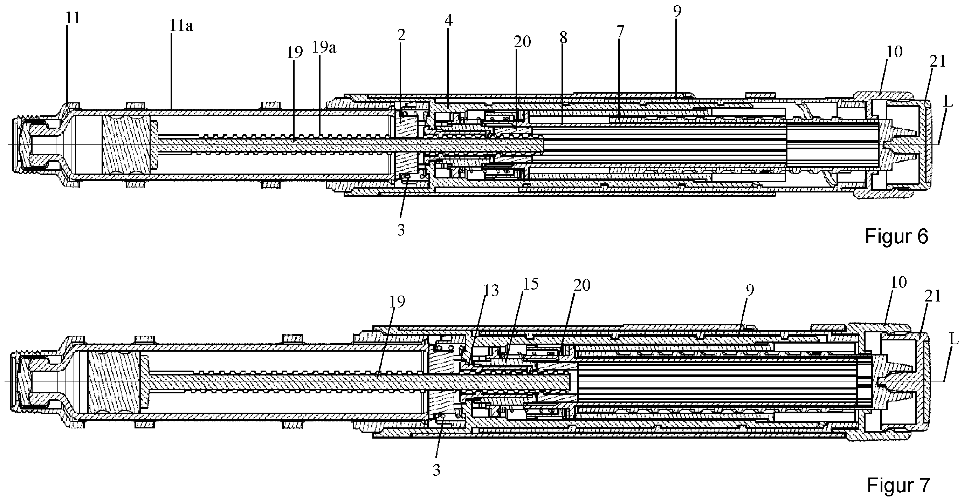

In Figur 6 wird die Vorrichtung in einem Zustand gezeigt, bei dem die in dem Produktbehälter 11a enthaltene, ausschüttbare Menge kleiner ist als die mit der Vorrichtung maximal einstellbare Dosis. Um zu verhindern, dass mit der Vorrichtung eine Dosis einstellbar ist, die größer ist als die in dem Produktbehälter 11a enthaltene, ausschüttbare Produktmenge, weist die Vorrichtung ein Begrenzungselement 20 auf, welches in dem gezeigten Beispiel hülsenförmig ist und ein Innengewinde 20b aufweist, welches in das Gewinde 19a der Kolbenstange 19 eingreift. Das Begrenzungselement 20 ist ferner verdrehgesichert und axial verschiebbar mit der Antriebshülse 8 verbunden, insbesondere in einem solchen Eingriff mit der Antriebshülse 8. Die Antriebshülse 8 kann z. B. hierfür an ihrem Umfang mindestens eine Längsnut aufweisen, in welche eine Abragung 20c am Außenumfang des Begrenzungselements 20 eingreift.

Das Begrenzungselement 20 weist ferner, insbesondere an seinem distalen Ende, einen Gegenanschlag 20a in der Gestalt einer Fläche auf, deren Flächennormale in Umfangsrichtung weist. Das Rotationsglied 13, 15, insbesondere die Kupplungshülse 15 weist einen Stoppanschlag in der Gestalt einer Fläche, deren Flächennormale in Umfangsrichtung weist, auf. Der entlang einer helixförmigen Kurve, insbesondere mit der Steigung des Gewindes 19a, bestehende Abstand zwischen dem Stoppanschlag 15a und dem Gegenanschlag 20a ist proportional zu der einstellbaren und/oder aus dem Produktbehälter ausschüttbaren Dosis oder Menge.

Das Begrenzungselement 20 ist vorzugsweise permanent drehfest mit dem Dosiseinstellelement 10 verbunden, so dass es die Drehungen bei den Dosier- und Ausschüttbewegungen des Dosiseinstellelements 10 mitmacht. In den Figuren 2a und 2b wird das Begrenzungselement 20 in Bezug auf die Kolbenstange 19 in einer Ausgangsposition oder Nulldosisposition, bei der die Dosis Null eingestellt ist, gezeigt. Wenn das Begrenzungselement 20 für die Dosiseinstellung in eine erste Drehrichtung für eine Dosiserhöhung relativ zu der Kolbenstange 19 gedreht wird, führt das drehfest mit dem Dosiseinstellelement 10 verbundene Begrenzungselement 20 ebenfalls eine Drehbewegung relativ zu der Kolbenstange 19 aus, wodurch das Begrenzungselement 20 relativ zu der Kolbenstange 19 und dem Gehäuse 4, 18 in distale Richtung geschraubt wird (Figur 3). Wird das Dosiseinstellelement 10 relativ zu dem Gehäuse 4, 18 für eine Dosiskorrektur oder - verringerung in die zweite Drehrichtung gedreht, wird das Begrenzungselement 20 relativ zu der Kolbenstage 19 und relativ zu dem Gehäuse 4, 18 in proximale Richtung geschraubt.

Wird das Dosiseinstellelement 10 für die Dosisausschüttung, d. h. bei betätigtem Betätigungselement 21, in die zweite Drehrichtung gedreht (Figur 4), wird das Begrenzungselement 20 relativ zu der Kolbenstange 19 zu dem proximalen Ende der Kolbenstange 19 hin geschraubt, wobei das Begrenzungselement 20 in Bezug auf das Gehäuse 4, 18 und/oder auf das Rotationsglied 13, 15 seine Position entlang der Längsachse L beibehält. Während der Dosisausschüttung führt das Begrenzungselement 20 lediglich eine Drehbewegung und keine Axialbewegung relativ zu dem Gehäuse 4, 18 aus. Vorzugsweise ist das Begrenzungselement 20 während der Dosisausschüttung drehfest in Bezug auf das Rotationsglied 13, 15. Das bedeutet, dass sich der Abstand zwischen dem Stoppanschlag 15a und dem Gegenanschlag 20a während der Dosisausschüttung nicht verändert. Am Ende der Ausschüttung, d. h., wenn in der Zeigeeinrichtung der Wert Null ablesbar ist, nimmt das Begrenzungselement 20 die gleiche Position in Bezug auf die Kolbenstange 19 ein, wie vor der Einstellung der ausgeschütteten Dosis (vgl. Figuren 2a, 2b mit Figur 5).

Wenn die in dem Produktbehälter 11a enthaltene Menge geringer ist als die maximal einstellbare Dosis, schlägt der Gegenanschlag 20a des Begrenzungselements 20 an dem Stoppanschlag 15a an, wenn das Dosiseinstellelement 10 in die erste Drehrichtung für eine Erhöhung der Dosis gedreht wird. Bei dem Versuch das Dosiseinstellelement 10 weiter in die erste Drehrichtung zu drehen, wird das Dosiseinstellelement 10 gegen eine Drehung in die erste Drehrichtung gesperrt aufgrund des an dem Rotationsglied 13, 15 anschlagenden Begrenzungselements 20. Eine Einstellung einer Dosis, welche die in dem Produktbehälter 11a enthaltene, ausschüttbare Produktmenge übersteigt, wird hierdurch verhindert.

In einer nicht gezeigten Alternative ist der Abstand zwischen Gegenanschlag 20a und Stoppanschlag 15b im Vergleich zu der gezeigten Ausführungsform verringert, d.h. das Begrenzungselement befindet sich in den Zeichnungen weiter links, wodurch die Drehung des Dosiseinstellelements 10 in die erste Drehrichtung früher verhindert wird. In dem Produktbehälter 11a verbleibt nach der Ausschüttung der letzten Dosis eine Restmenge, welche die Differenz aus der Nennfüllmenge des Produktbehälters 11a und der ausgeschütteten Menge bzw. Ausschüttmenge ist. In der Alternative wird die Einstellung einer Dosis verhindert, welche bewirken würde, dass in dem Produktbehälter 11a weniger als die Restmenge der Nennfüllmenge verbleibt.

Zum Ausschütten der in Figur 6 eingestellten Dosis wird das Betätigungselement 21 wie oben beschrieben betätigt und das Dosiseinstellelement 10 mit dem Antriebshub in das Gehäuse zurückgeschraubt. Am Ende der Ausschüttung (Figur 7) nimmt das Begrenzungselement 20 in Bezug auf die Kolbenstange 19 wieder die gleiche Position ein, wie in den Nulldosispositionen der vorhergehenden Ausschüttungen (Figuren 2a, 2b, Figur 5).

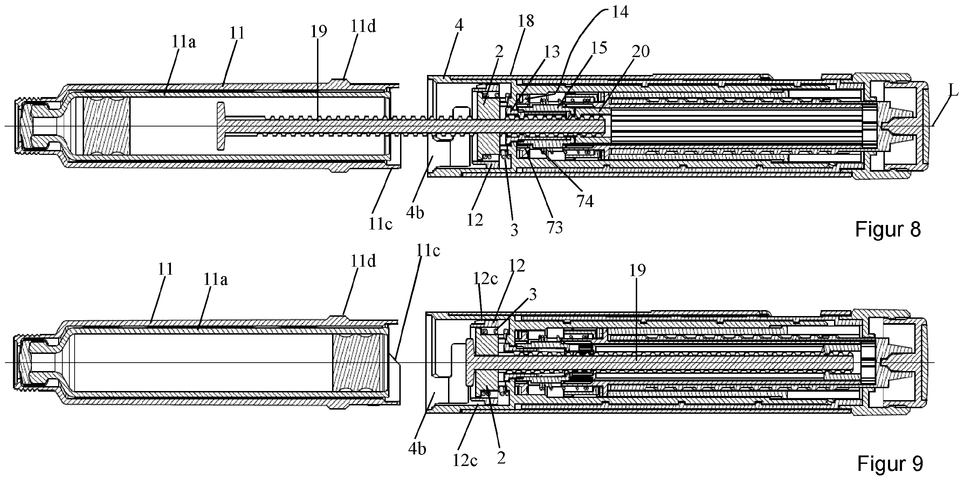

Bei Ausführungen, bei denen ein entleerter Produktbehälter 11a (Figur 8) gegen einen neuen Produktbehälter 11a (Figur 9) ausgetauscht werden kann, kann der hülsenförmige Produktbehälterhalter 11, der den Produktbehälter 11a aufnimmt, von der Antriebs- und Dosiervorrichtung gelöst und entfernt werden. Aus dem entfernten Produktbehälterhalter 11 wird der Produktbehälter 11a entnommen. In den Produktbehälterhalter 11 wird ein neuer Produktbehälter 11a über das proximale Ende des Produktbehälterhalters 11 eingeschoben. Der Produktbehälterhalter 11 wird dann an der Antriebs- und Dosiervorrichtung mittels eines Bajonettverschlusses oder eines Steck-Dreh-Verschlusses befestigt.

Der Produktbehälterhalter 11 weist an seinem äußeren Umfang einen Nocken 11b und einen insbesondere ringförmig über den Umfang umlaufenden Kragen 11d auf. Das Gehäuse 4, 18, insbesondere der Mechanikhalter 4 weist einen Bajonettschlitz 4b auf, der einen sich in etwa parallel zur Längsachse L erstreckenden Abschnitt und einen sich in Umfangsrichtung erstreckenden Abschnitt aufweist, der sich insbesondere an den sich in Axialrichtung erstreckenden Abschnitt anschließt. Im befestigten Zustand (siehe z. B. Figur 7) befindet sich der Nocken 11b des Produktbehälterhalters 11 in dem sich in Umfangsrichtung erstreckenden Abschnitt des Bajonettschlitzes 4b. Der Kragen 11d liegt an dem distalen Ende des Gehäuses 4, 18, insbesondere des Mechanikhalters 4 an. Zum Lösen des Produktbehälterhalters 11 wird dieser in eine Drehrichtung relativ zu dem Gehäuse 4, 18 gedreht, so dass sich der Nocken 11b in den sich entlang der Längsachse L erstreckenden Abschnitt des Bajonettschlitzes 4b bewegt. Wenn der Nocken 11b dort angekommen ist, kann der Produktbehälterhalter 11 mit einer Bewegung entlang der Längsachse L aus der Antriebs- und Dosiervorrichtung herausgezogen und entfernt werden. Zum Befestigen des Produktbehälterhalters 11 an der Antriebs- und Dosiervorrichtung wird dieser Vorgang umgekehrt ausgeführt. Der Produktbehälterhalter 11 wird in die entgegengesetzte Richtung in das distale Ende der Antriebs- und Dosiervorrichtung eingesteckt, wobei der Nocken 11b in den sich entlang der Längsachse L erstreckenden Abschnitt des Bajonettschlitzes 4b gesteckt wird. Durch Drehen des Produktbehälterhalters 11 relativ zu dem Gehäuse 4, 18 wird der Nocken in dem sich in Umfangsrichtung erstreckenden Abschnitt des Bajonettschlitzes 4b bewegt, wodurch der Produktbehälterhalter 11 an dem Gehäuse fixiert wird. Insbesondere kann hierdurch der Kragen 11d gegen das distale Ende des Mechanikhalters 4 gedrückt werden.

Die in dem Produktbehälterhalter 11 aufnehmbaren Produktbehälter 11a, die auch als Karpule bezeichnet werden, sind in der Regel aus Kunststoff oder Glas gefertigt. Die Länge des Produktbehälters 11a unterliegt verhältnismäßig hohen Toleranzen, wie z. B. im Bereich mehrerer Zehntel mm. Die in den Figuren 1 bis 10b gezeigte Vorrichtung weist eine Einrichtung auf, die sicherstellt, dass der Produktbehälter 11a trotz hoher Herstellungstoleranzen stets fest in dem Produktbehälterhalter 11 aufgenommen ist. Zu dieser Einrichtung gehört das Führungsglied 2, welches mittels des proximalen Endes des Produktbehälters 11a entlang der Längsachse L bzw. relativ zu dem Gehäuse 4, 18 verschiebbar ist. Auf das Führungsglied 2 wirkt eine Feder 3, die auch als Toleranzausgleichsfeder bezeichnet werden kann. Die Feder 3 stützt sich mit ihrem distalen Ende an dem Führungsglied 2 und mit ihrem proximalen Ende an dem Gehäuse 4, 18, insbesondere an dem Mechanikhalter 4 ab. Beim Befestigen des Produktbehälterhalters 11 an der Antriebs- und Dosiervorrichtung wirkt das proximale Ende gegen das Führungsglied 2, wodurch das Führungsglied 2 in proximale Richtung relativ zu dem Gehäuse 4, 18 verschoben und die Feder 3 gespannt wird. Insbesondere liegt das proximale Ende des Produktbehälters 11a an dem Führungsglied 2 an. Die Feder 3 drückt den Produktbehälter 11a in einen axialfesten Sitz mit dem Produktbehälterhalter 11. Durch die federnde Anordnung des Führungsglieds 2 können Längentoleranzen des Produktbehälters 11a ausgeglichen werden. Zwischen der Feder 3 und dem proximalen Ende des Produktbehälters 11a ist das Führungsglied 2 angeordnet.

Beim Lösen des Produktbehälterhalters 11 von der Antriebs- und Dosiervorrichtung wird das Führungsglied 2 entlang der Längsachse L und relativ zu dem Gehäuse 4, 18 in distale Richtung verschoben aufgrund der vorgespannten Feder 3.

Wie aus den Figuren 8 und 9 erkennbar ist, muss die Kolbenstange 19 für das Befestigen eines neuen Produktbehälters 11a in ihre Ausgangsposition zurückgesetzt werden.

Die unidirektionale Kupplung 70 ist hierfür aus einem aktiven Schaltzustand, in welcher sie die Drehung des Rotationsglieds 13, 15 in die erste Drehrichtung blockiert, in einen inaktiven Schaltzustand, in der sie die Drehung des Rotationsglieds 13, 15 in die erste Drehrichtung zulässt, schaltbar.

Die Kupplung 70 weist eine dritte Kupplungsstruktur 73 an dem Kupplungsring 14 und eine vierte Kupplungsstruktur 74 an dem Rotationsglied 13, 15, und insbesondere an der Kupplungshülse 15 auf. Wenn der Produktbehälterhalter 11 an der Antriebs- und Dosiervorrichtung befestigt ist, sind die dritte Kupplungsstruktur 73 und die vierte Kupplungsstruktur 74 in einem verdrehgesicherten Eingriff, so dass das Rotationsglied 13, 15 drehfest, d. h. in beide Drehrichtungen drehfest mit dem Kupplungsring 14 gekoppelt ist. Der Produktbehälterhalter 11 ist so mit der dritten Kupplungsstruktur 73 gekoppelt, dass die dritte Kupplungsstruktur 73 beim Lösen des Produktbehälterhalters 11 von der Antriebs- und Dosiervorrichtung aus dem Eingriff mit der vierten Kupplungsstruktur 74 bewegt wird, insbesondere mit einer Bewegung entlang der Längsachse L und relativ zu dem Gehäuse 4, 18 in distale Richtung. Die Kupplung 70 ist dann in ihrem inaktiven Schaltzustand. Der Produktbehälterhalter 11 ist so mit der dritten Kupplungsstruktur 73 gekoppelt, dass beim Befestigen des Produktbehälterhalters 11 an der Antriebs- und Dosiervorrichtung die dritte Kupplungsstruktur 73 in den Eingriff mit der vierten Kupplungsstruktur 74 bewegt wird. Die vierte Kupplungsstruktur 74 führt hierbei eine Bewegung entlang der Längsachse L und relativ zu dem Gehäuse 4, 18 in proximale Richtung aus. Insbesondere kann die dritte Kupplungsstruktur 73, insbesondere der Kupplungsring 14, an dem die dritte Kupplungsstruktur gebildet ist, gegen die Kraft der Feder 5 in proximale Richtung verschoben werden, wodurch die Feder 5 gespannt wird. Insbesondere kann die Feder 5 die dritte Kupplungsstruktur 73, insbesondere den Kupplungsring 14, in distale Richtung bewegen, wenn der Produktbehälterhalter 11 von dem Gehäuse 4, 18 gelöst wird. Die Feder 5 kann sich hierfür an dem Kupplungsring 14 abstützen, insbesondere mit ihrem distalen Ende.

Die dritte Kupplungsstruktur 73 ist vorzugsweise eine Innenverzahnung, wobei die vierte Kupplungsstruktur 74 vorzugsweise eine Außenverzahnung ist.

Die dritte Kupplungsstruktur 73, insbesondere der Kupplungsring 14, ist über das Schaltglied 12, welches in Bezug auf das Gehäuse 4, 18 drehfest und entlang der Längsachse L verschiebbar ist, mit dem Produktbehälterhalter 11 gekoppelt. In dem gezeigten Beispiel ist das Schaltglied 12 derart mit dem Produktbehälterhalter 11 gekoppelt, dass eine Drehung des Produktbehälterhalters 11 relativ zu dem Gehäuse 4, 18 eine Axialbewegung des Schaltglieds 12 entlang der Längsachse L bewirkt. Das Schaltglied 12 wird in distale Richtung bewegt, wenn der Produktbehälterhalter 11 in eine Drehrichtung gedreht wird, die das Lösen des Produktbehälterhalters 11 von dem Gehäuse 4, 18 bewirkt. Das Schaltglied 12 wird in proximale Richtung bewegt, wenn der Produktbehälterhalter 11 in eine Drehrichtung gedreht wird, die eine Befestigung des Produktbehälterhalters 11 an dem Gehäuse 4, 18 bewirkt. Insbesondere kann der Produktbehälterhalter 11 bei einer Drehbewegung relativ zu dem Gehäuse 4, 18 und dem Schaltglied 12 an dem Schaltglied 12 abgleiten. Der Produktbehälterhalter 11 weist an seinem proximalen Ende z. B. ein Aktivierungselement 11c auf, welches eine abgeschrägte Getriebefläche umfasst, die an dem Schaltglied 12 abgleitet, wenn der Produktbehälterhalter 11 relativ zu dem Schaltglied 12 gedreht wird, wodurch die Axialbewegung des Schaltglieds 12 bewirkt wird. Vorzugsweise weist das Schaltglied 12 eine Ausnehmung 12c auf, die an die Form des Aktivierungselements 11c angepasst ist, wobei das Aktivierungselement 11c beim Einstecken der Abragung 11b in den axialen Abschnitt des Bajonettschlitzes 4b in die Ausnehmung 12c gesteckt wird. Beim folgenden Drehen des Produktbehälterhalters 11 relativ zu dem Schaltglied 12 wird das Aktivierungselement 11c aus der Ausnehmung 12c herausgedreht, wobei das Schaltglied 12 entlang der Längsachse L bewegt wird. Beim Drehen des Produktbehälterhalters 11 in die entgegengesetzte Drehrichtung für das Lösen des Produktbehälterhalters 11 von der Antriebs- und Dosiervorrichtung wird das Aktivierungselement 11c in die Ausnehmung 12c hinein gedreht, wobei das Schaltglied 12 aufgrund der in distale Richtung wirkenden Kraft der vorgespannten Feder 5 in distale Richtung bewegt wird.

Wie bereits erwähnt, befindet sich die Kupplung 70 in einem inaktiven Schaltzustand, wenn der Produktbehälterhalter 11 von der Antriebs- und Dosiervorrichtung gelöst ist (Figur 8). Die Kolbenstange 19 kann dann in die Antriebs- und Dosiervorrichtung zurückgesetzt oder zurückgeschoben werden, insbesondere mittels der Muskelkraft des Benutzers. Hierbei ist die Kolbenstange 19 drehfest in Bezug auf das Gehäuse 4, 18, wobei die Kolbenstange 19 mit einer Axialbewegung entlang der Längsachse L in das Gehäuse 4, 18 zurückgeschoben wird. Hierdurch wird das Begrenzungselement 20 mitgenommen, wobei das Begrenzungselement 20 dreh- und axialfest in Bezug auf die Kolbenstange 19 ist. Ferner wird beim Zurücksetzen der Kolbenstange 19 das Rotationsglied 13, 15 in seine erste Drehrichtung gedreht, da - wie gesagt - die Kupplung 70 in ihrem inaktiven Schaltzustand ist.

Nachdem die Kolbenstange 19 zurückgesetzt wurde (Figur 9), kann der Produktbehälterhalter 11 an der Antriebs- und Dosiervorrichtung - wie beschrieben - befestigt werden.

Die Antriebs- und Dosiervorrichtung aus den Figuren 1 bis 10b weist ferner einen Mechanismus auf, der bei der Dosiseinstellung, insbesondere während der Dosiserhöhung und der Dosisverringerung ein akustisches und/oder taktiles Signal ausgibt, welches auch als "Klick" bezeichnet werden kann, und/oder diskrete Winkelschritte für das Dosiseinstellelement 10 vorgibt, um die das Dosiseinstellelement 10 relativ zu dem Gehäuse 4, 18 drehbar ist. Der Mechanismus umfasst insbesondere eine Ratschenhülse 16, einen Ratschenring 17 und die Antriebshülse 8. An einem nach außen abragenden ringförmigen Kragen der Antriebshülse 8 ist eine in distale Richtung weisende erste Signalkupplungsstruktur 91 angebracht, die in diesem Beispiel eine sägezahnförmige Verzahnung ist. Der Ratschenring 17 weist eine zweite Signalkupplungsstruktur 92 auf, die in proximale Richtung weist und in einem Eingriff mit der ersten Signalkupplungsstruktur 91 ist. Der Ratschenring 17 weist ferner eine dritte Signalkupplungsstruktur 93 auf, die in distale Richtung weist und in einem Eingriff mit einer vierten Signalkupplungsstruktur 94, die in proximale Richtung weist und an der Ratschenhülse 16 angeordnet ist, eingreift. Die Signalkupplungsstrukturen 91 bis 94 sind jeweils sich über den Umfang des jeweiligen Bauteils erstreckende sägezahnförmige Verzahnungen. Die einzelnen Sägezähne der Verzahnung sind mit einer Winkelteilung voneinander beabstandet, welche die diskreten Winkelschritte für das Dosiseinstellelement 10 vorgibt. Wenn mit der Antriebs- und Dosiervorrichtung beispielsweise Dosen in Schritten von 1 I.U. eingestellt werden sollen, ist die Winkelteilung der Sägezähne so, dass ein diskreter Winkelschritt des Dosiseinstellelements 10 der Drehung des Dosiseinstellelements 10 für 1 I.U. entspricht. Dementsprechend kann während der Drehung des Dosiseinstellelements je einem diskreten Winkelschritt ein akustisches bzw. taktiles Signal ausgegeben werden. Der Vollständigkeit halber sei angemerkt, dass die Winkelteilung auch für Winkelschritte größer oder kleiner als 1 I.U. sein kann, wie z.B. 0,5 oder 2 oder 5 I.U. Die Ratschenhülse 16 greift verdrehgesichert und entlang der Längsachse L verschiebbar in das Gehäuse 4, 18, insbesondere in den Mechanikhalter 4 ein, wenn das Betätigungselement 21 unbetätigt ist und wird aus diesem Eingriff entlang der Längsachse L verschoben, wenn das Betätigungselement 21 betätigt wird, so dass die Ratschenhülse 16 relativ zu dem Gehäuse 4, 18 drehbar ist. Die Ratschenhülse 16 weist eine Außenverzahnung 16a auf, die in eine Innenverzahnung 4d am Innenumfang des Mechanikhalters 4 eingreift, wodurch die Ratschenhülse 16 relativ zu dem Gehäuse 4, 18 verdrehgesichert ist. Wenn das Betätigungselement 21 betätigt wird, wird die Außenverzahnung 16a entlang der Längsachse L aus dem Eingriff mit der Innenverzahnung 4d verschoben, so dass die Ratschenhülse 16 relativ zu dem Gehäuse 4, 18 drehbar ist. Mit anderen Worten ist die Ratschenhülse 16 während der Dosiseinstellung drehfest in Bezug auf das Gehäuse 4, 18 und während der Produktausschüttung relativ zu dem Gehäuse 4, 18 drehbar.

Der genannte Mechanismus dient in der gezeigten Ausführungsform ferner dazu, dem Antriebsglied 8 einen gewissen Drehwiderstand gegen Verdrehung in die zweite Drehrichtung bereitzustellen, um während des Betätigungshubs des Betätigungselements 21, d.h. wenn das Betätigungselement noch nicht in seiner betätigten Position ist, eine frühzeitige Verdrehung des Antriebsglieds 8 zu verhindern. Der Drehwiderstand ist vorzugsweise (minimal) größer als das durch das Verschieben des Betätigungselements 21, insbesondere aufgrund des Gewindeeingriffs zwischen Dosierhülse 7 und Koppelglied 6, auf das Antriebsglied 8 bewirkte Drehmoment. Wenn beim Betätigen die Zwischenposition des Betätigungselements 21, die insbesondere weiter unten näher beschrieben wird, überschritten wurde, fällt der beschriebene Drehwiderstand weg.

Optional kann, da die Ausschüttkupplung 81, 82 bereits geschlossen ist, die unidirektionale Kupplung 70 dem Antriebsglied 8 einen gewissen Drehwiderstand gegen Verdrehung in die zweite Drehrichtung bereitstellen, um während des Betätigungshubs des Betätigungselements 21, d.h. wenn das Betätigungselement 21 noch nicht in seiner betätigten Position ist, eine frühzeitige Verdrehung des Antriebsglieds 8 in die zweite Drehrichtung zu verhindern. Wenn das Betätigungselement 21 in seiner betätigten Position ist und vom Verwender weiter in distale Richtung gedrückt wird, wird der Drehwiderstand der unidirektionalen Kupplung 70 überwunden, wodurch das Antriebsglied 8 und das Rotationsglied 13, 15 in die zweite Drehrichtung gedreht werden.

Bei der Dosiserhöhung, d. h. der Drehung des Dosiseinstellelements 10 in die erste Drehrichtung und bei der Dosisverringerung, d. h. bei Drehung des Dosiseinstellelements 10 in die zweite Drehrichtung wird die Antriebshülse 8 jeweils relativ zu der Ratschenhülse 16 verdreht. Durch den zwischen der ersten Signalkupplungsstruktur 91 und der vierten Signalkupplungsstruktur 94 angeordneten Ratschenring 17 wird bewirkt, dass sich der Ratschenring 17 beim Erhöhen der Dosis relativ zu einem aus Ratschenhülse 16 und Antriebshülse 8 mitdreht und zu dem anderen aus Ratschenhülse 16 und Antriebshülse 8 nicht mitdreht. Beim Verringern der Dosis dreht sich der Ratschenring 17 relativ zu dem einen aus Ratschenhülse 16 und Antriebshülse 8 nicht mit und zu dem anderen aus Ratschenhülse 16 und Antriebshülse 8 mit. In dem gezeigten Beispiel wird beim Erhöhen der Dosis der Ratschenring 17 relativ zu der Ratschenhülse 16 gedreht, wobei der Ratschenring 17 die Drehung der Antriebshülse 8 mitmacht. Bei der Dosisverringerung wird die Ratschenhülse 8 relativ zu dem Ratschenring 17 gedreht, wobei der Ratschenring 17 relativ zu der Ratschenhülse 16 nicht gedreht wird. Da während der Dosiserhöhung und -verringerung immer Signalkupplungsstrukturen 91 bis 94 aneinander abgleiten, werden die so genannten Klicks erzeugt. Die Feder 5 stützt sich mit ihrem proximalen Ende an der Ratschenhülse 16 ab und hält die Ratschenhülse 16, den Ratschenring 17 und die Antriebshülse 8 in einem Ratscheneingriff. Ferner setzt die Feder 5 - wie beschrieben - das Betätigungselement 21 in seine Ausgangsposition zurück. Durch Drücken des Betätigungselements 21 wird die Ratschenhülse 16 bzw. die Außenverzahnung 16a durch Verschieben der Antriebshülse 8 und des Ratschenrings 17 in distale Richtung aus dem drehfesten Eingriff mit dem Gehäuse 4, 18 bzw. der Innenverzahnung 4d verschoben, wodurch der Klickerzeugungsmechanismus während der Produktausschüttung inaktiviert ist. Während der Verschiebung des Betätigungselements 21, d.h. wenn das Betätigungselement 21 zwischen der betätigten Position und der unbetätigten Position, d.h. in eine Zwischenposition verschoben ist, ist es bevorzugt, dass die Ausschüttkupplung 81, 82 geschlossen und die Kupplung 16a, 4d geschlossen sind. Hierdurch wird sichergestellt, dass während des Betätigungshubs des Betätigungselements 21 die Kupplung 16a, 4d erst dann geöffnet wird, wenn die Ausschüttkupplung 81, 82 bereits geschlossen ist. Dadurch wird sichergestellt, dass keine unbeabsichtigte Verstellung der eingestellten Dosis während des Betätigungshubs stattfindet.

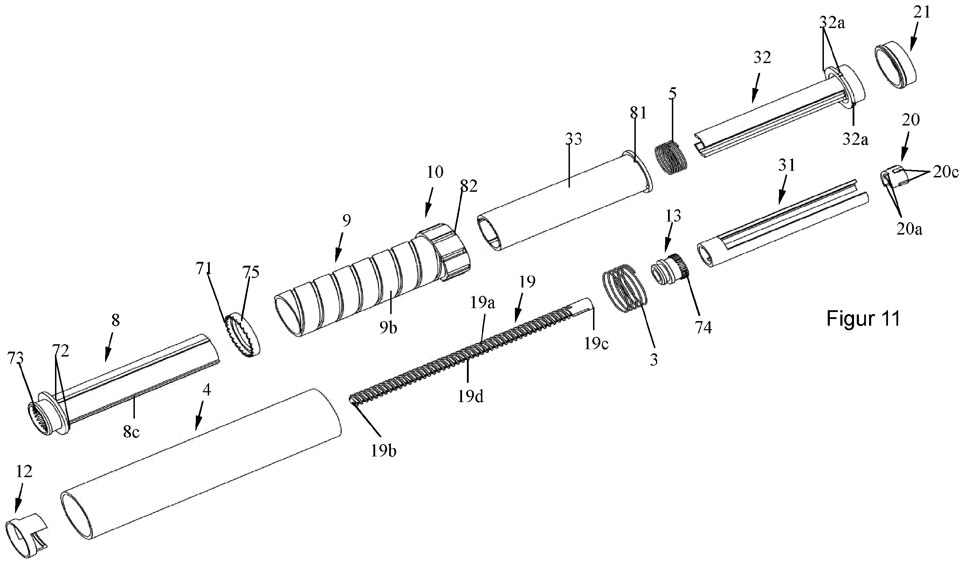

In der in den Figuren 11 bis 18 gezeigten zweiten Ausführungsform der Erfindung weist die Antriebs- und Dosiervorrichtung ein hülsenförmiges Gehäuse 4 auf, welches gleichzeitig als Mechanikhalter dient. Das Gehäuse 4 weist ein distales Ende, an dem eine Produktbehälteraufnahme mit einem Produktbehälter (nicht gezeigt) befestigt werden kann und ein proximales Ende, an dem ein drehbares Dosiseinstellelement 10 angeordnet ist, auf. Das Gehäuse 4 weist ein Innengewinde auf, welches in ein Außengewinde einer Dosisanzeigetrommel 9 eingreift, die dreh- und axialfest mit dem Dosiseinstellelement 10 verbunden ist. Obwohl nicht gezeigt, weist das Gehäuse 4 eine Zeigeeinrichtung und der Außenumfang der Dosisanzeigetrommel 9 eine Dosisskala wie bei der ersten Ausführungsform auf. Für die Dosiseinstellung ist das Dosiseinstellelement 10 durch Drehung in eine erste Drehrichtung relativ zu dem Gehäuse 4 aus dem proximalen Ende des Gehäuses 4 herausschraubbar und für eine Dosiskorrektur und eine Dosisausschüttung in das distale Ende des Gehäuses 4 hineinschraubbar, wobei das Dosiseinstellelement 10 eine Drehung in die zweite Drehrichtung ausführt.

Die Antriebs- und Dosiervorrichtung weist eine Kolbenstange 19 auf, die ein Außengewinde 19a und mindestens eine sich parallel zur Längsrichtung erstreckende Führung, insbesondere Führungsnut, aufweist. In die Führung greift ein Gehäuseabschnitt oder ein drehfest mit dem Gehäuse 4 verbundenes Teil ein. Die Kolbenstange 19 ist relativ zu dem Gehäuse 4 permanent drehfest und entlang der Längsachse L der Kolbenstange 19, die der Längsachse L der Antriebs- und Dosiervorrichtung entspricht, relativ zu dem Gehäuse 4 verschiebbar. In das Außengewinde 19a der Kolbenstange 19 greift ein Innengewinde eines Rotationsglieds 13 ein, wobei das Rotationsglied 13 axialfest und drehbar mit dem Gehäuse 4 verbunden ist, insbesondere in einem solchen Eingriff mit dem Gehäuse 4 ist. Die Kolbenstange 19 weist ein distales Ende 19b und ein proximales Ende 19c auf.

Das Rotationsglied 13 ist über eine Rücksetzkupplung 73, 74 drehfest mit einer Antriebshülse 8 verbunden, wenn an der Antriebs- und Dosiervorrichtung eine Produktbehälteraufnahme und/oder ein Produktbehälter befestigt ist. Die Rücksetzkupplung 73, 74 weist eine Kupplungsstruktur 74 in der Gestalt einer sich über den Außenumfang des Rotationsglieds 13 erstreckenden Außenverzahnung auf. Die Antriebshülse 8 weist eine sich über ihren Innenumfang erstreckende Innenverzahnung auf, welche die Kupplungsstruktur 73 bildet. Wenn die Kupplungsstruktur 73 mit der Kupplungsstruktur 74 in einem Eingriff ist, sind die Antriebshülse 8 und das Rotationsglied 13 drehfest verbunden. Die Antriebshülse 8 ist mit einer sie umgebenden Kupplungshülse 33 drehfest und axial verschiebbar verbunden, wie z. B. über Abragungen am Außenumfang der Antriebshülse 8, die in Führungen am Innenumfang der Kupplungshülse 33 eingreifen. Die Kupplungshülse 33 weist an ihrem proximalen Ende einen nach außen abragenden Kragen auf, der eine erste Ausschüttkupplungsstruktur 81 aufweist, die in dem gezeigten Beispiel mehrere in distale Richtung weisende Zähne sind. Die erste Ausschüttkupplungsstruktur 81 ist Teil einer Ausschüttkupplung 81, 82, die ferner eine zweite Ausschüttkupplungsstruktur 82 aufweist, die von dem Dosiseistellelement 10 oder/und der Dosisanzeigetrommel 9 gebildet wird. Die zweite Ausschüttkupplungsstruktur 82 weist eine Vielzahl über den Umfang umlaufende Zähne auf. Am proximalen Ende der Antriebs- und Dosiervorrichtung ist ein Betätigungselement 21 in der Gestalt eines Betätigungsknopfs gebildet, der durch Drücken in distale Richtung um einen Betätigungshub relativ zu dem Dosiseinstellelement 10 in distale Richtung in eine betätigte Position verschiebbar ist. Zwischen der Kupplungshülse 33 und dem Betätigungselement 21 ist eine Rücksetzfeder 5 angeordnet, welche das Betätigungselement 21 beim Loslassen in die unbetätigte Position zurücksetzt und beim Betätigen bzw. Drücken des Betätigungselements 21 gespannt wird.

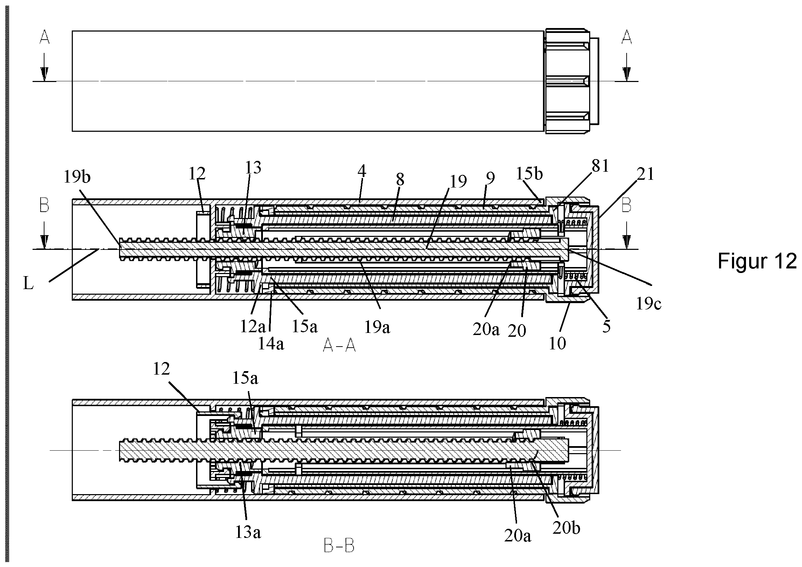

Während der Dosiseinstellung, d. h. Dosiserhöhung und -verringerung ist die Antriebshülse 8 drehfest in Bezug auf das Gehäuse 4. Gleiches gilt für die Kupplungshülse 33. Wird das Dosiseinstellelement 10 aus der in Figur 12 gezeigten Position in eine erste Drehrichtung für eine Dosiserhöhung gedreht, wird es aus dem proximalen Ende des Gehäuses 4 herausgeschraubt, wobei die Kupplungsstruktur 82 der Ausschüttkupplung 81, 82 an der Kupplungsstruktur 81 entlanggleitet, wobei die Kupplungsstruktur 81 bzw. die Kupplungshülse 33 eine leicht entlang der Längsachse L oszillierende Bewegung ausführt. Die Kraft der Feder 5 ist hierbei so gewählt, dass sie die Kupplungsstruktur 81 nur so leicht in den Eingriff mit der Kupplungsstruktur 82 drückt, dass eine Drehung des Dosiseinstellelements 10 relativ zu dem Gehäuse 4 bewirkt, dass die Zähne der Kupplungsstruktur 81 aus dem Eingriff mit der Kupplungsstruktur 82 gedrückt werden, ohne dass die Kupplungshülse 33 die Drehbewegung des Dosiseinstellelements 10 mitmacht.

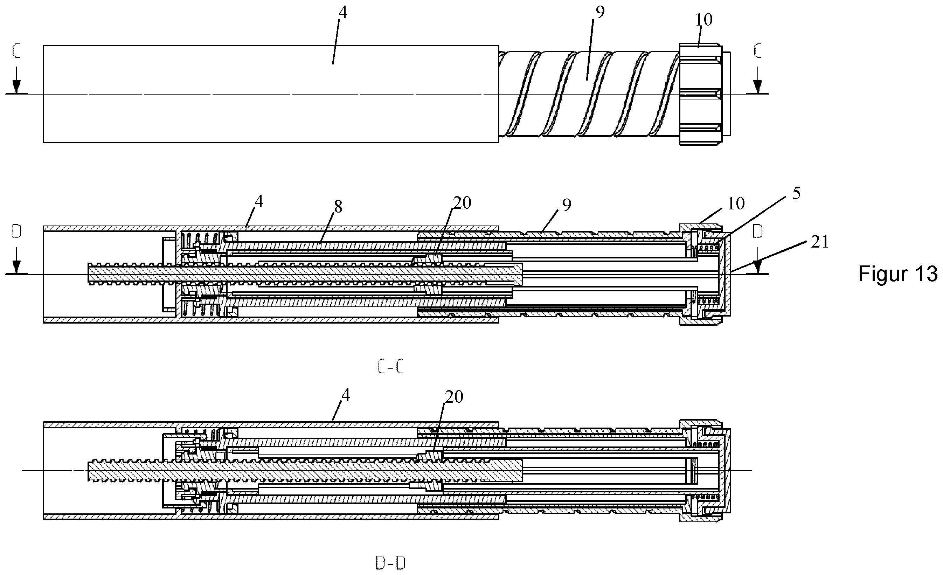

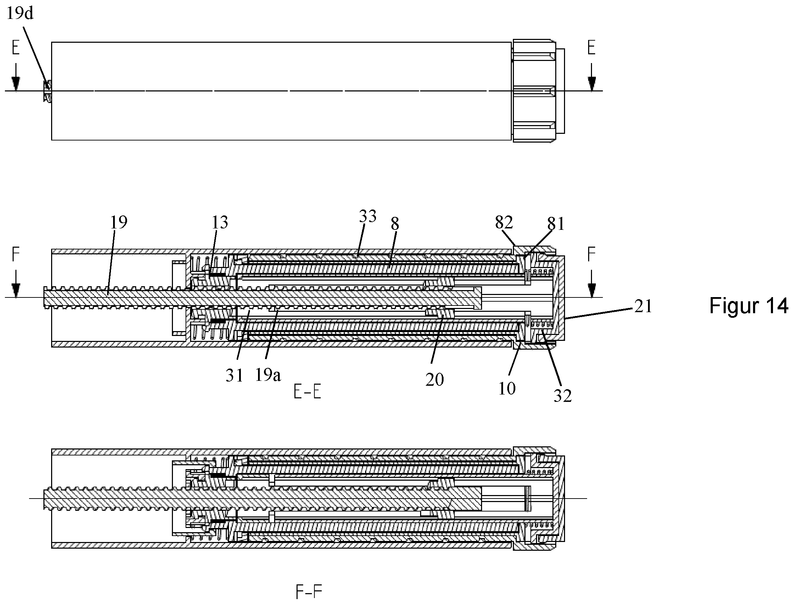

Wenn die gewünschte Dosis eingestellt ist (Figur 13) wird das Betätigungsglied 21 gedrückt (Figur 14), wobei durch die Betätigung des Betätigungselements 21 die Kupplungsstrukturen 81, 82 in einem drehfesten Eingriff gehalten werden, wodurch die Kupplungshülse 33 die Drehbewegung des Dosiseinstellelements 10 mitmacht, wenn das Dosiseinstellelement 10 in das Gehäuse zurückgeschraubt wird (vgl. Figuren 13 und 14) und seinen Antriebshub ausführt. Allgemein ausgedrückt ist das Dosiseinstellelement 10 bei betätigtem Betätigungsglied 21 drehfest mit dem Rotationsglied 13 gekoppelt, nämlich über die Ausschüttkupplung 81, 82, die permanent drehfeste Verbindung zwischen der Kupplungshülse 33 und der Antriebshülse 8 und der zumindest zeitweise, insbesondere bei befestigten Produktbehälterhalter 11, drehfesten Verbindung zwischen der Antriebshülse 8 und dem Rotationsglied 13.

Aufgrund der Drehung des Rotationsglieds 13 in die zweite Drehrichtung relativ zu der Kolbenstange 19 wird die Kolbenstange 19 um ihren Ausschütthub in distale Richtung verschoben.

Die Antriebs- und Dosiervorrichtung der zweiten Ausführungsform weist ebenfalls eine Einrichtung zur Verhinderung der Einstellung einer Dosis, welche die in dem Produktbehälter enthaltene Produktmenge übersteigt, auf. Dieser Mechanismus umfasst ein Begrenzungselement 20, welches die Kolbenstange 19 ringförmig umgibt und ein Innengewinde aufweist, welches in das Außengewinde der Kolbenstange 19 eingreift. Das Begrenzungselement 20 ist insbesondere permanent drehfest mit dem Dosiseinstellelement 10 verbunden, insbesondere über eine Koppeleinrichtung 31, 32. Die Koppeleinrichtung 31, 32 weist eine erste Koppelhülse 31 und eine zweite Koppelhülse 32 auf. Die erste Koppelhülse 31 und die zweite Koppelhülse 32 greifen so ineinander, dass sie relativ zueinander drehfest und axial verschiebbar sind. Die zweite Koppelhülse macht bei der Dosiseinstellung und -ausschüttung die Schraubbewegungen des Dosiseinstellelements 10 mit. Das Begrenzungselement 20 greift in die erste und/oder zweite Koppelhülse 31, 32 drehfest und axial verschiebbar ein. Hierfür können die erste Koppelhülse und/oder die zweite Koppelhülse 32 jeweils eine Führungsnut für eine am äußeren Umfang des Begrenzungselements 20 gebildete Abragung aufweisen, die in die Führungsnut eingreift. Die zweite Koppelhülse 32 ist drehfest und um in etwa den Betätigungshub des Betätigungselements 21 axial verschiebbar mit dem Dosiseinstellelement 10 verbunden. Hierfür weist die zweite Koppelhülse 32 mindestens eine Abragung 32a auf, welche in eine Führung, die sich parallel zur Längsachse L der Vorrichtung erstreckt, eingreift. Die Feder 5 stützt sich insbesondere an der zweiten Koppelhülse 32 ab. Vorzugsweise wird ein an der zweiten Koppelhülse 32 gebildeter Flansch gegen die Kupplungshülse 33, insbesondere deren proximales Ende, gedrückt, wenn das Betätigungselement 21 betätigt ist, wodurch die Kupplungsglieder 81, 82 in ihren zueinander drehfesten Eingriff gehalten werden.

Das Betätigungselement 21 ist vorzugsweise in Bezug auf das Dosiseinstellelement 10 und/oder die zweite Koppelhülse 32 frei drehbar. Hierfür kann das Betätigungselement 21 eine im Bereich des Rotationszentrums angeordnete Kontaktfläche mit möglichst geringem Durchmesser aufweisen, die in einem Kontakt mit einer am proximalen Ende der zweiten Koppelhülse 32 gebildeten Wand ist. Hierdurch wird eine Verringerung der Reibung bewirkt, wenn eine Drehung zwischen der Koppelhülse 32a und dem Betätigungselement 21 stattfindet.

Wenn das Dosiseinstellelement 10 für eine Dosiserhöhung in die erste Drehrichtung gedreht wird, wird das Begrenzungselement 20 ebenfalls in die erste Drehrichtung relativ zu der Kolbenstange 19 gedreht, wodurch das Begrenzungselement 20 an der Kolbenstange 19 entlang und relativ zu dem Gehäuse 4 in Richtung distalem Ende 19b der Kolbenstange 19 geschraubt wird. Wird das Dosiseinstellelement 10 in die zweite Drehrichtung für eine Dosisverringerung gedreht, wird das Begrenzungselement 20 ebenfalls in die zweite Drehrichtung relativ zu der Kolbenstange 19 gedreht, wodurch sich das Begrenzungselement 20 an der Kolbenstange 19 entlang und relativ zu dem Gehäuse 4 in Richtung proximalem Ende 19c der Kolbenstange 19 schraubt.

Wird das Betätigungselement 21 für eine Produktausschüttung betätigt, und das Dosiseinstellelement 10 um den Antriebshub in das Gehäuse 4 zurück gedrückt bzw. geschraubt, wird das Begrenzungselement 20 an der Kolbenstange 19 in Richtung proximalem Ende der Kolbenstange 19 geschraubt, wobei es in Bezug auf das Gehäuse 4 axial fest steht und eine Drehbewegung ausführt. Der Grund hierfür ist, dass die Kolbenstange 19 in Ausschüttrichtung verschoben wird.

Am Ende der Ausschüttung (Figur 14) nimmt das Begrenzungselement 20 die gleiche Position in Bezug auf die Kolbenstange 19 ein, wie in der Nulldosisposition vor der Einstellung und Ausschüttung der Dosis. Die Kinematik des Begrenzungselements 20 ist gleich zu der der ersten Ausführungsform.

Ebenfalls wie bei der ersten Ausführungsform weist das Begrenzungselement 20 mindestens einen Gegenanschlag 20a auf, der für den Anschlag an einen Stoppanschlag 15a vorgesehen ist, der an dem Rotationsglied 13 gebildet ist.

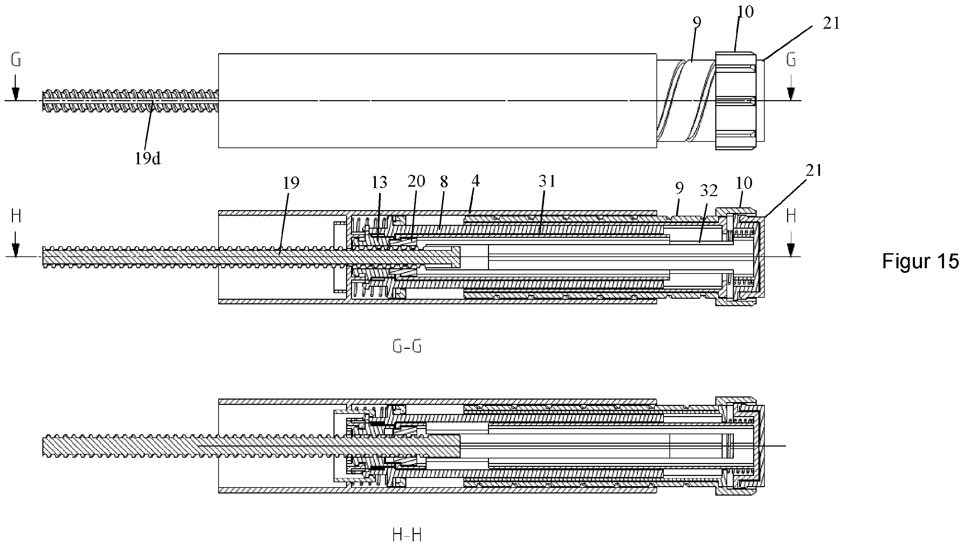

In Figur 15 wird die Situation gezeigt, bei der das Begrenzungselement 20 die Einstellung einer Dosis verhindert, welche die in dem Produktbehälter enthaltene Produktmenge übersteigt. Durch Drehung des Dosiseinstellelements 10 in die erste Drehrichtung wird ein Abstand zwischen dem Gegenanschlag 20a und dem Stoppanschlag 15a verringert. Der Abstand ist proportional zu der in dem Produktbehälter enthaltenen Produktmenge. Schlägt das Begrenzungselement 20 an dem Rotationsglied 13 an, wird eine Drehung des Begrenzungselements 10 in die erste Drehrichtung verhindert oder gesperrt.

Auch diese Ausführungsform kann - wie die erste Ausführungsform - durch, wie z.B. bei der Montage, das Verringern des Abstandes zwischen dem Stoppanschlag 15a und dem Gegenanschlag 20a so modifiziert werden, dass die Einstellung einer Dosis verhindert wird, welche bewirken würde, dass in dem Produktbehälter weniger als die Restmenge der Nennfüllmenge verbleibt.

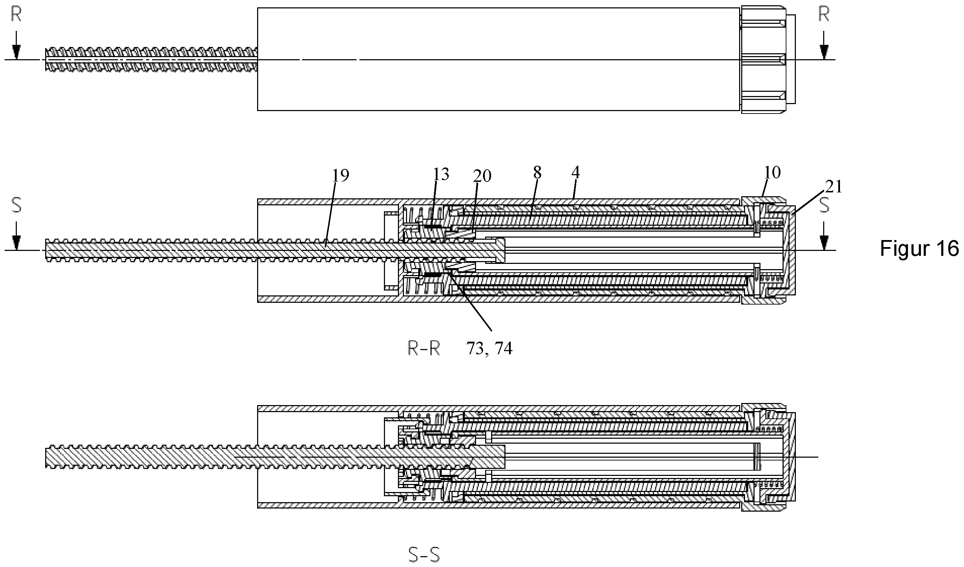

Figur 16 zeigt die Antriebs- und Dosiervorrichtung nach der Ausschüttung der in Figur 15 eingestellten Dosis. Eine Drehung des Dosiseistellelements 10 in die erste Drehrichtung ist nicht mehr möglich.

Zwischen dem Rotationsglied und dem Gehäuse 4 ist eine z. B. als Ratsche ausgestaltete, unidirektionale Kupplung 71, 72 angeordnet, die in ihrem aktivierten Schaltzustand die Drehung des Rotationsglieds 13 relativ zu der Kolbenstange 19 nur in die zweite Drehrichtung zulässt und in die erste Drehrichtung sperrt. Durch Entfernen des Produktbehälters oder des Produktbehälterhalters von der Antriebs- und Dosiervorrichtung wird die unidirektionale Kupplung 71, 72 inaktiviert, insbesondere durch Lösen des Kupplungseingriffs der Kupplungsstrukturen 73 und 74 und/oder der die unidirektionale Kupplung 71, 72 bildenden Kupplungsstrukturen 71, 72.

Die unidirektionale Kupplung 71, 72 weist eine erste Kupplungsstruktur 71 auf, die an dem Gehäuse 4 oder einem gehäusefesten Element, hier einem ringförmigen Element 75, gebildet ist. Die zweite Kupplungsstruktur 72 ist an der Antriebshülse 8 angeordnet, und weist mindestens einen sägezahnförmigen, insbesondere in proximale Richtung abragenden Zahn auf, der, wenn er in die erste Kupplungsstruktur 71 eingreift, die Drehung des Antriebsglieds 8 nur in die zweite Drehrichtung, aber nicht in die erste Drehrichtung zulässt.

Der Produktbehälter oder der Produktbehälterhalter (nicht gezeigt) sind so mit der Antriebshülse 8 verbunden, dass die Antriebshülse 8 beim Lösen des Produktbehälters von der Antriebs- und Dosiervorrichtung relativ zu dem Gehäuse 4 in distale Richtung verschoben wird und beim Befestigen des Produktbehälters an der Antriebs- und Dosiervorrichtung relativ zu dem Gehäuse 4 in proximale Richtung verschoben wird. Zwischen dem Produktbehälterhalter und der Antriebshülse 8 oder zwischen dem Produktbehälter und der Antriebshülse 8 ist ein Schaltglied 12 angeordnet, welches durch den Produktbehälterhalter oder den Produktbehälter selbst betätigt wird, so dass das Schaltglied 12 entlang der Längsachse L verschoben wird. Schaltglied 12 und Antriebshülse 8 sind so miteinander verbunden, dass die Antriebshülse 8 die Bewegungen des Schaltglieds 12 entlang der Längsachse L mitmacht.

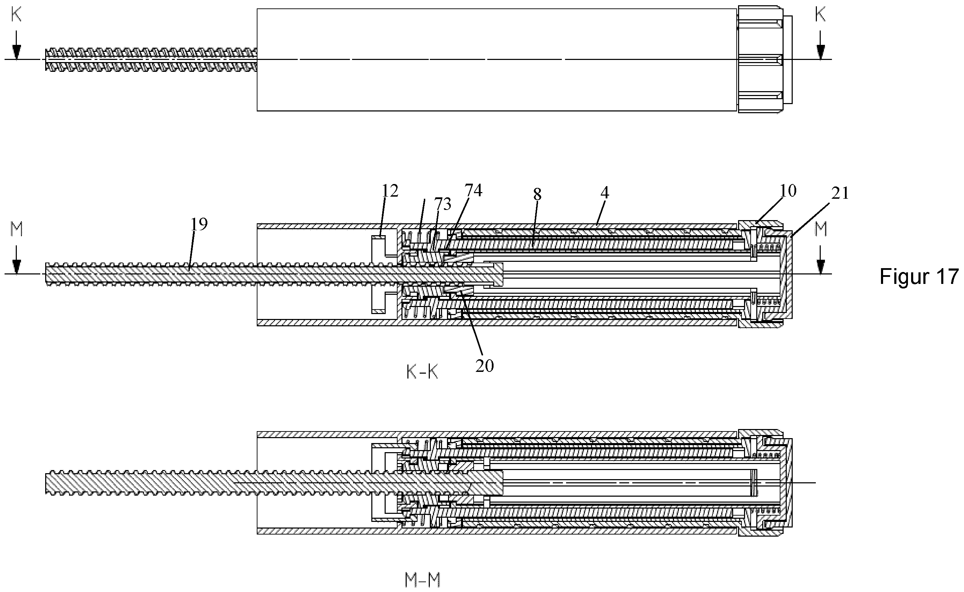

Wenn der Produktbehälterhalter von der Antriebs- und Dosiervorrichtung gelöst ist, sind die Kupplungen 71, 72 und/oder 73, 74 geöffnet, wodurch das Rotationsglied 13 relativ zu dem Gehäuse 4 in die erste Drehrichtung drehbar ist (Figur 17). Durch Ausüben einer in die proximale Richtung wirkenden Kraft auf die Kolbenstange 19 kann die Kolbenstange 19 in die Antriebs- und Dosiervorrichtung zurückgeschoben werden, wodurch das Rotationsglied 13 sich relativ zu dem Gehäuse 4 in die erste Drehrichtung dreht. Das Begrenzungselement 20 wird hierbei von der Kolbenstange 19 mitgenommen und führt bei der Rücksetzbewegung keine Bewegung relativ zu der Kolbenstange 19 aus.

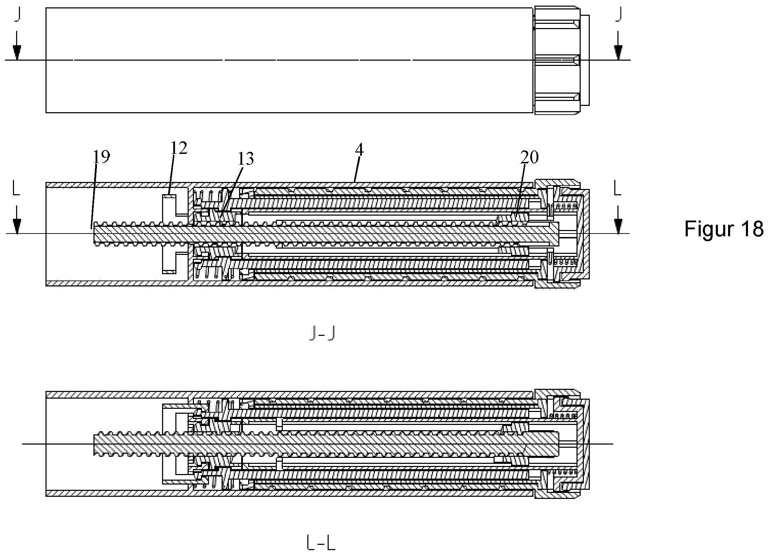

Nachdem die Kolbenstange 19 vollständig zurückgesetzt ist (Figur 18) kann der Produktbehälter bzw. der Produktbehälterhalter wieder an der Antriebs- und Dosiervorrichtung befestigt werden, wodurch das Schaltglied 12 und somit das Antriebsglied 8 in proximale Richtung relativ zu dem Gehäuse 4 verschoben werden. Hierbei werden die Kupplungen 71, 72 bzw. 73, 74 wieder geschlossen, so dass eine Drehung des Rotationsglieds 13 nur noch in die zweite Drehrichtung, aber nicht mehr in die erste Drehrichtung möglich ist.

**Bezugszeichenliste**

| | | | |
|---|---|---|---|
| 1 | Abdeckkappe | 15a | Stoppanschlag |
| | | 16 | Ratschenhülse |
| 2 | Führungsglied | 16a | Außenverzahnung |
| 2a | Eingriffsabschnitt | | |
| | | 17 | Ratschenring |
| 3 | Feder | | |
| | | 18 | Gehäuse/äußere Gehäusehülse |
| 4 | Mechanikhalter | 18a | Zeigeeinrichtung/Fenster |
| 4a | Gewinde | | |
| 4b | Bajonettschlitz | 19 | Kolbenstange |
| 4c | Nulldosisanschlag (alternativ) | 19a | Außengewinde |
| 4d | Innenverzahnung | 19b | Flansch/distales Ende |
| | | 19c | proximales Ende |
| 5 | Feder | 19d | Längsnut/Führungsnut |
| | | | |
| 6 | Koppelglied/Koppelhülse | 20 | Begrenzungselement |
| 6a | Rippe | 20a | Gegenanschlag |
| 6b | Innengewinde / Innengewindesegment | 20b | Innengewinde |
| | | 20c | Abragung |
| 7 | Dosierhülse | 21 | Betätigungselement/Betätigungs-knopf |
| 7a | Rippe | | |
| 7b | Gewinde | 31 | erste Koppelhülse |
| 7c | Führungsschlitz | 32 | zweite Koppelhülse |
| 7d | Nulldosisanschlag | 32a | Abragung |
| 7e | Maximaldosisanschlag | | |
| | | 33 | Kupplungshülse |
| 8 | Antriebshülse | | |
| 8c | Führungsrippen | 71 | erste (Ausschütt-)Kupplungsstruktur/ sägezahnförmige Verzahnung |
| 9 | Dosisanzeigetrommel | 72 | zweite (Ausschütt-)Kupplungsstruktur/ sägezahnförmige Verzahnung |
| 9b | Dosisskala | | |
| 10 | Dosiseinstellelement | 73 | dritte (Ausschütt-)Kupplungsstruktur/ Innenverzahnung |
| 10a | Eingriffsglied | 74 | vierte (Ausschütt-)Kupplungsstruktur/ Außenverzahnung |
| 10b | Abragung | | |
| | | 75 | Kupplungsring/ringförmiges Element |
| 11 | Produktbehälterhalter/-aufnahme | | |
| 11a | Produktbehälter/Karpule | 81 | erste Ausschüttkupplungsstruktur/ Innenverzahnung |
| 11b | Bajonettabragung | | |
| 11c | Aktivierungselement | 82 | zweite Ausschüttkupplungsstruktur/ Außenverzahnung |
| 11d | Kragen | | |
| | | | |
| 12 | Schaltglied | 91 | erste Signalkupplungsstruktur/ sägezahnförmige Verzahnung |
| 12c | Ausnehmung | | |
| | | 92 | zweite Signalkupplungsstruktur/ sägezahnförmige Verzahnung |
| 13 | Gewindemutter | | |
| 13a | Innengewinde | 93 | dritte Signalkupplungsstruktur/ sägezahnförmige Verzahnung |
| 14 | Kupplungsring | | |
| | | 94 | vierte Signalkupplungsstruktur/ sägezahnförmige Verzahnung |
| 15 | Kupplungshülse | | |

## Patentansprüche

1. Antriebs- und Dosiervorrichtung für eine Injektionsvorrichtung, wobei an der Antriebs- und Dosiervorrichtung ein Produktbehälter (11a) befestigt oder befestigbar ist, die Antriebs- und Dosiervorrichtung umfassend:
a) ein Gehäuse (4, 18),
b) eine Kolbenstange (19), die ein distales Ende (19b), ein proximales Ende (19c) und ein Gewinde (19a) aufweist und für eine Produktausschüttung in Bezug auf das Gehäuse (4, 18) in eine Ausschüttrichtung bewegbar ist,
c) ein Rotationsglied (13, 15), welches operativ, insbesondere verdrehfest, mit einem Stoppanschlag (15a) verbunden ist und in das Gewinde (19a) der Kolbenstange (19) eingreift, wobei eine Drehung des Rotationsglieds (13, 15) relativ zu der Kolbenstange (19) bewirkt, dass die Kolbenstange (19) relativ zu dem Gehäuse (4, 18) und mit ihrem distalen Ende (19b) voran in die Ausschüttrichtung bewegt wird,
d) ein Dosiseinstellelement (10), welches zur Erhöhung einer aus dem Produktbehälter (11a) auszuschüttenden Dosis relativ zu dem Gehäuse (4, 18) und/oder der Kolbenstange (19) in eine erste Drehrichtung drehbar ist,
e) ein Begrenzungselement (20), welches einen Gegenanschlag (20a) und ein Gewinde (20b), welches in das Gewinde (19a) der Kolbenstange (19) oder ein weiteres Gewinde der Kolbenstange (19) eingreift, aufweist, wobei das Dosiseinstellelement (10) so mit dem Begrenzungselement (20) gekoppelt ist, dass eine Drehung des Dosiseinstellelements (10) relativ zu der Kolbenstange (19) in die erste Drehrichtung eine Drehung des Begrenzungselements (20) relativ zu der Kolbenstange (19) bewirkt, wodurch das Begrenzungselement (20) zu dem distalen Ende (19b) der Kolbenstange (19) hin geschraubt und der Gegenanschlag (20a) zu dem Stoppanschlag (15a) hin bewegt wird,
f) wobei, wenn der Gegenanschlag (20a) an dem Stoppanschlag (15a) anschlägt, das Begrenzungselement (20) die Einstellung einer Dosis oder die Drehung des Dosiseinstellelements (10) in die erste Drehrichtung verhindert.

2. Antriebs- und Dosiervorrichtung nach Anspruch 1, **dadurch gekennzeichnet, dass** das Dosiseinstellelement (10) zur Verringerung einer eingestellten Dosis in eine zweite, der ersten Drehrichtung entgegengesetzte Drehrichtung drehbar ist und die Drehung des Dosiseinstellelements (10) relativ zu der Kolbenstange (19) in die zweite Drehrichtung eine Drehung des Begrenzungselements (20) relativ zu der Kolbenstange (19) bewirkt, wodurch das Begrenzungselement (20) zu dem proximalen Ende (19c) der Kolbenstange (19) hin geschraubt und der Gegenanschlag (20a) von dem Stoppanschlag (15a) weg bewegt wird.

3. Antriebs- und Dosiervorrichtung nach einem der vorhergehenden Ansprüche, **gekennzeichnet durch** ein Betätigungselement (21), wobei die Antriebs- und Dosiervorrichtung angepasst ist, dass eine Betätigung, insbesondere das Drücken, des Betätigungselements (21) durch den Benutzer bewirkt, dass das Rotationsglied (13, 15) relativ zu dem Gehäuse (4, 18) und der Kolbenstange (19) gedreht wird, wodurch sich die Kolbenstange (19) relativ zu dem Gehäuse (4, 18) in die Ausschüttrichtung bewegt, und dass das Begrenzungselement (20) zu dem proximalen Ende (19c) der Kolbenstange (19) hin geschraubt wird.

4. Antriebs- und Dosiervorrichtung nach einem der vorhergehenden Ansprüche, **dadurch gekennzeichnet, dass** zwischen dem Gehäuse (4, 18) und dem Rotationsglied (13, 15) eine unidirektionale Kupplung (71, 72, 73, 74) angeordnet ist, welche die Drehung des Rotationsglieds (13, 15) in eine Drehrichtung, welche die Bewegung der Kolbenstange (19) in die Ausschüttrichtung bewirkt, zulässt und in die entgegengesetzte Drehrichtung nicht zulässt.

5. Antriebs- und Dosiervorrichtung nach dem vorhergehenden Anspruch, **dadurch gekennzeichnet, dass** die unidirektionale Kupplung (71, 72, 73, 74) zwischen einem aktivierten Schaltzustand und einem inaktivierten Schaltzustand hin und her schaltbar ist, wobei die unidirektionale Kupplung (71, 72, 73, 74) in ihrem aktivierten Schaltzustand die Drehung des Rotationsglieds (13, 15) in eine Drehrichtung, welche die Bewegung der Kolbenstange (19) in die Ausschüttrichtung bewirkt, zulässt und in die entgegengesetzte Drehrichtung nicht zulässt, und in ihrem inaktivierten Schaltzustand die Drehung des Rotationsglieds (13, 15) in die entgegengesetzte Drehrichtung zulässt.

6. Antriebs- und Dosiervorrichtung nach dem vorhergehenden Anspruch, **gekennzeichnet durch** einen Produktbehälterhalter (11), in dem der Produktbehälter (11a) aufnehmbar oder aufgenommen ist und der am distalen Ende des Gehäuses (4, 18) lösbar befestigt oder befestigbar ist, wobei zwischen dem Produktbehälterhalter (11) oder dem Produktbehälter (11a) und der unidirektionalen Kupplung (71, 72, 73, 74) ein Schaltglied (12) angeordnet ist, welches durch das Befestigen des Produktbehälterhalters (11) oder des Produktbehälters (11a) relativ zu dem Gehäuse (4, 18), insbesondere in die proximale Richtung, verschiebbar ist und die unidirektionale Kupplung (71, 72, 73, 74) in ihrem aktivierten Schaltzustand schaltet und durch Lösen des Produktbehälterhalters (11) oder des Produktbehälters (11a), insbesondere in die distale Richtung, verschiebbar ist und die unidirektionale Kupplung (71, 72, 73, 74) in ihren inaktivierten Schaltzustand schaltet.

7. Antriebs- und Dosiervorrichtung nach einem der zwei vorhergehenden Ansprüche, **dadurch gekennzeichnet, dass** die Gewindesteigung der ineinandergreifenden Gewinde (19a, 13a) des Rotationsglieds (13, 15) und der Kolbenstange (19) so groß ist, dass keine Selbsthemmung auftritt, wenn die Kolbenstange (19) bei inaktivierter unidirektionaler Kupplung (71, 72, 73, 74) entgegen die Ausschüttrichtung zurückgesetzt wird, wodurch das Rotationsglied (13, 15) eine Drehung in die entgegengesetzte Drehrichtung ausführt.

8. Antriebs- und Dosiervorrichtung nach einem der vorhergehenden Ansprüche, **gekennzeichnet durch** eine Dosisanzeigehülse (9), die zumindest drehfest, vorzugsweise auch axialfest, mit dem Dosiseinstellelement (10) gekoppelt ist, wobei die Dosisanzeigehülse (9) über ihren Umfang eine wendelförmig angeordnete Dosisskala (9b) aufweist, wobei das Gehäuse (4, 18) eine Zeigeeinrichtung (18a), insbesondere ein Fenster aufweist, in dem derjenige Wert der Dosisskala (9b) ablesbar ist, welcher der eingestellten Dosis entspricht, wobei die Dosisanzeigetrommel (9) mittels Drehung des Dosiseinstellelements (10) eine Schaubbewegung relativ zu dem Gehäuse (4, 18) ausführt, so dass sich die Dosisskala (9b) an der Zeigeeinrichtung (18a) entlang bewegt.

9. Antriebs- und Dosiervorrichtung nach dem vorhergehenden Anspruch, **dadurch gekennzeichnet, dass** die Dosisanzeigetrommel (9) zwischen einer Nulldosisposition und einer Maximaldosisposition hin und her schraubbar ist, wobei ein Nulldosisanschlag (4c; 7d) in der Nulldosisposition der Dosisanzeigetrommel (9) die Drehung der Dosisanzeigetrommel (9) in die Drehrichtung, welche eine Verringerung der Dosis bewirkt, verhindert und in die Drehrichtung, welche eine Erhöhung der Dosis bewirkt, zulässt, und ein Maximaldosisanschlag (7e) in der Maximaldosisposition der Dosisanzeigetrommel (9) die Drehung der Dosisanzeigetrommel (9) in die Drehrichtung, welche eine Erhöhung der Dosis bewirkt, verhindert und in die Drehrichtung, welche eine Verringerung der Dosis bewirkt, zulässt.

10. Antriebs- und Dosiervorrichtung nach dem vorhergehenden Anspruch, **dadurch gekennzeichnet, dass** der Gegenanschlag (20a) des Begrenzungselements (20) an dem Stoppanschlag (15a) anschlägt und das Begrenzungselement (20) die Drehung der Dosisanzeigetrommel (9) in die Drehrichtung, welche eine Erhöhung der Dosis bewirkt, verhindert, wenn die Dosisanzeigetrommel (9) nicht in ihrer Maximaldosisposition ist und/oder entweder wenn die Menge des Produkts in dem Produktbehälter (11a) geringer als die Dosis in der Maximaldosisposition ist oder der in dem Produktbehälter (11a) enthaltene restliche Teil der Ausschüttmenge geringer als die Dosis in der Maximaldosisposition ist.

11. Antriebs- und Dosiervorrichtung nach einem der vorhergehenden Ansprüche, **dadurch gekennzeichnet, dass** das Begrenzungselement (20) drehfest mit dem Dosiseinstellelement (10) gekoppelt ist, wodurch das Begrenzungselement (20) während der Dosiseinstellung und der Verabreichung der eingestellten Dosis an der Kolbenstange (19) entlanggeschraubt wird.

12. Antriebs- und Dosiervorrichtung nach einem der vorhergehenden Ansprüche, **gekennzeichnet durch** ein zum Ausschütten der eingestellten Dosis betätigbares Betätigungselement (21) und eine Ausschüttkupplung (81, 82), wobei die Ausschüttkupplung (81, 82) mittels Betätigen, insbesondere Drücken, des Betätigungselement (21) durch den Benutzer der Vorrichtung geschlossen und durch Loslassen des Betätigungselement (21) geöffnet wird, wobei das Rotationsglied (13, 15) verdrehfest mit einer Dosisanzeigetrommel (9) und/oder dem Dosiseinstellelement (10) gekoppelt ist, wenn die Ausschüttkupplung (81, 82) geschlossen ist, und die Dosisanzeigetrommel (9) und/oder das Dosiseinstellelement (10) relativ zu dem Rotationsglied (13, 15) drehbar ist, wenn die Ausschüttkupplung (81, 82) geöffnet ist.

13. Antriebs- und Dosiervorrichtung nach einem der vorhergehenden Ansprüche, **dadurch gekennzeichnet, dass** das Begrenzungselement (20) permanent oder zumindest während der Dosiseinstellung und des Ausschüttens der eingestellten Dosis drehfest mit einer Dosisanzeigetrommel (9) und/oder dem Dosiseinstellelement (10) gekoppelt ist.

14. Antriebs- und Dosisvorrichtung nach einem der vorhergehenden Ansprüche, **dadurch gekennzeichnet, dass** kinematisch zwischen dem Dosiseinstellelement (10) und dem Begrenzungselement (20) mindestens eine Zwischenhülse (7; 30, 31) angeordnet ist, in die das Begrenzungselement (20) verdrehfest und axial verschiebbar eingreift und die verdrehfest mit dem Dosiseinstellelement (10) gekoppelt ist.

15. Antriebs- und Dosiervorrichtung nach einem der vorhergehenden Ansprüche, **dadurch gekennzeichnet, dass** das Begrenzungselement (20) in der Nulldosisposition unmittelbar nach einer Ausschüttung einer eingestellten Dosis in Bezug auf die Kolbenstange (19) die gleiche Position einnimmt wie in der Nulldosisposition unmittelbar vor der Einstellung der Dosis für diese Ausschüttung.

16. Injektionsvorrichtung zur Ausschüttung eines vorzugsweise flüssigen Produkts, umfassend die Antriebs- und Dosiervorrichtung nach einem der vorhergehenden Ansprüche und einen Produktbehälter (11a), der eine Nennfüllmenge des Produkts enthält, wobei mit der Injektionsvorrichtung nur eine Ausschüttmenge der Nennfüllmenge ausschüttbar ist und eine Restmenge der Nennfüllmenge nicht ausschüttbar ist,
g) wobei das Begrenzungselement (20) während einer Drehung des Dosiseinstellelements (10) in die erste Drehrichtung bei der Erhöhung der Dosis eine Bewegung zu dem Stoppanschlag (15a) hin ausführt, wodurch ein sich entlang einer helixförmigen Kurve erstreckender Abstand, der direkt proportional zu der Ausschüttmenge ist, zwischen dem Stoppanschlag (15a) und dem Begrenzungselement (20) verringert wird, und wobei das Begrenzungselement (20) während der Ausschüttung der eingestellten Dosis in Bezug auf den Stoppanschlag (15a) stillsteht, wobei wenn das Begrenzungselement (20) an dem Stoppanschlag (15a) anschlägt, die Einstellung einer Dosis verhindert wird, deren Ausschüttung auch die Ausschüttung zumindest einer Teilmenge der Restmenge bewirken würde oder wodurch die Einstellung einer Dosis verhindert wird, welche bewirken würde, dass in dem Produktbehälter (11a) weniger als die Restmenge der Nennfüllmenge verbleibt.

17. Verfahren zur Herstellung einer Injektionsvorrichtung nach Anspruch 16, wobei eine Nennfüllmenge für einen Produktbehälter (11a) für die Injektionsvorrichtung festgelegt wird, die Injektionsvorrichtung einen Stoppanschlag (15a) und ein Begrenzungselement (20) aufweist, und das Begrenzungselement (20) in ein Gewinde (19a) eingreift, das eine helixförmige Kurve vorgibt, **dadurch gekennzeichnet, dass** die Injektionsvorrichtung so montiert wird, dass nur eine Ausschüttmenge der Nennfüllmenge ausschüttbar ist und eine Restmenge der Nennfüllmenge nicht ausschüttbar ist, wobei dies dadurch erfolgt, dass das Begrenzungselement (20) in Bezug auf den Stoppanschlag (15a) mit einem sich entlang der helixförmigen Kurve erstreckenden Abstand, der direkt proportional zu der Ausschüttmenge ist, angeordnet wird.

## Claims

1. A driving and dosing device for an injection device, wherein a product container (11a) is or can be fastened to the driving and dosing device, the driving and dosing device comprising:
a) a housing (4, 18);
b) a piston rod (19) which comprises a distal end (19b), a proximal end (19c) and a thread (19a) and can be moved in relation to the housing (4, 18) in a delivery direction for delivering product;
c) a rotational member (13, 15) which is operatively connected, in particular rotationally fixed, to a stopping abutment (15a) and engages with the thread (19a) of the piston rod (19), wherein rotating the rotational member (13, 15) relative to the piston rod (19) causes the piston rod (19) to be moved relative to the housing (4, 18), with its distal end (19b) to the fore, in the delivery direction;
d) a dosage setting element (10) which can be rotated relative to the housing (4, 18) and/or piston rod (19) in a first rotational direction in order to increase a dosage to be delivered from the product container (11a);
e) a limiting element (20) which comprises a counter abutment (20a) and a thread (20b) which engages with the thread (19a) of the piston rod (19) or with another thread of the piston rod (19), wherein the dosage setting element (10) is coupled to the limiting element (20) such that rotating the dosage setting element (10) relative to the piston rod (19) in the first rotational direction causes the limiting element (20) to be rotated relative to the piston rod (19), thus screwing the limiting element (20) towards the distal end (19b) of the piston rod (19) and moving the counter abutment (20a) towards the stopping abutment (15a);
f) wherein when the counter abutment (20a) abuts against the stopping abutment (15a), the limiting element (20) prevents a dosage from being set or the dosage setting element (10) from being rotated in the first rotational direction.

2. The driving and dosing device according to Claim 1, **characterised in that** the dosage setting element (10) can be rotated in a second rotational direction, opposite to the first rotational direction, in order to reduce a dosage set, and rotating the dosage setting element (10) relative to the piston rod (19) in the second rotational direction causes the limiting element (20) to be rotated relative to the piston rod (19), thus screwing the limiting element (20) towards the proximal end (19c) of the piston rod (19) and moving the counter abutment (20a) away from the stopping abutment (15a).

3. The driving and dosing device according to any one of the preceding claims, **characterised by** an activating element (21), wherein the driving and dosing device is adapted such that when the activating element (21) is activated, in particular pressed, by the user, this causes the rotational member (13, 15) to be rotated relative to the housing (4, 18) and piston rod (19), thus moving the piston rod (19) relative to the housing (4, 18) in the delivery direction, and causes the limiting element (20) to be screwed towards the proximal end (19c) of the piston rod (19).

4. The driving and dosing device according to any one of the preceding claims, **characterised in that** a unidirectional coupling (71, 72, 73, 74) is arranged between the housing (4, 18) and the rotational member (13, 15) and allows the rotational member (13, 15) to rotate in a rotational direction which causes the piston rod (19) to be moved in the delivery direction and does not allow it to rotate in the opposite rotational direction.

5. The driving and dosing device according to the preceding claim, **characterised in that** the unidirectional coupling (71, 72, 73, 74) can be switched back and forth between an activated switching state and an inactivated switching state, wherein in its activated switching state, the unidirectional coupling (71, 72, 73, 74) allows the rotational member (13, 15) to rotate in a rotational direction which causes the piston rod (19) to be moved in the delivery direction and does not allow it to rotate in the opposite rotational direction, and in its inactivated switching state allows the rotational member (13, 15) to rotate in the opposite rotational direction.

6. The driving and dosing device according to the preceding claim, **characterised by** a product container holder (11) in which the product container (11a) is or can be accommodated and which is or can be detachably fastened to the distal end of the housing (4, 18), wherein a switching member (12): is arranged between the product container holder (11) or product container (11a) and the unidirectional coupling (71, 72, 73, 74); can be shifted relative to the housing (4, 18), in particular in the proximal direction, by fastening the product container holder (11) or product container (11a) and switches the unidirectional coupling (71, 72, 73, 74) to its activated switching state; and can be shifted, in particular in the distal direction, by releasing the product container holder (11) or product container (11a) and switches the unidirectional coupling (71, 72, 73, 74) to its inactivated switching state.

7. The driving and dosing device according to any one of the preceding two claims, **characterised in that** the thread pitch of the mutually engaging threads (19a, 13a) of the rotational member (13, 15) and piston rod (19) is large enough that self-locking does not occur when the piston rod (19) is reset, counter to the delivery direction, while the unidirectional coupling (71, 72, 73, 74) is inactivated, whereby the rotational member (13, 15) performs a rotation in the opposite rotational direction.

8. The driving and dosing device according to any one of the preceding claims, **characterised by** a dosage indicating sleeve (9) which is coupled to the dosage setting element (10) such that it is at least rotationally fixed and preferably also axially fixed, wherein the dosage indicating sleeve (9) comprises a helically arranged dosage scale (9b) over its circumference, wherein the housing (4, 18) comprises an indicating device (18a), in particular a window, in which the value on the dosage scale (9b) which corresponds to the dosage set can be read off, wherein the dosage indicating drum (9) performs a helical movement relative to the housing (4, 18) by means of a rotation of the dosage setting element (10), such that the dosage scale (9b) is moved along the indicating device (18a).

9. The driving and dosing device according to the preceding claim, **characterised in that** the dosage indicating drum (9) can be screwed back and forth between a zero dosage position and a maximum dosage position, wherein in the zero dosage position of the dosage indicating drum (9), a zero dosage abutment (4c; 7d) prevents the dosage indicating drum (9) from rotating in the rotational direction which causes a reduction in the dosage and allows it to rotate in the rotational direction which causes an increase in the dosage, and in the maximum dosage position of the dosage indicating drum (9), a maximum dosage abutment (7e) prevents the dosage indicating drum (9) from rotating in the rotational direction which causes an increase in the dosage and allows it to rotate in the rotational direction which causes a reduction in the dosage.

10. The driving and dosing device according to the preceding claim, **characterised in that** the counter abutment (20a) of the limiting element (20) abuts against the stopping abutment (15a), and the limiting element (20) prevents the dosage indicating drum (9) from rotating in the rotational direction which causes an increase in the dosage when the dosage indicating drum (9) is not in its maximum dosage position and/or either when the amount of the product in the product container (11a) is less than the dosage in the maximum dosage position or the residual portion of the delivery amount contained in the product container (11a) is less than the dosage in the maximum dosage position.

11. The driving and dosing device according to any one of the preceding claims, **characterised in that** the limiting element (20) is coupled, rotationally fixed, to the dosage setting element (10), thus screwing the limiting element (20) along the piston rod (19) while the dosage is set and while the dosage set is administered.

12. The driving and dosing device according to any one of the preceding claims, **characterised by** an activating element (21), which can be activated in order to deliver the dosage set, and a delivery coupling (81, 82), wherein the delivery coupling (81, 82) is closed by means of the activating element (21) being activated, in particular pressed, by the user of the device and is opened by releasing the activating element (21), wherein the rotational member (13, 15) is coupled, rotationally fixed, to a dosage indicating drum (9) and/or the dosage setting element (10) when the delivery coupling (81, 82) is closed, and the dosage indicating drum (9) and/or dosage setting element (10) can be rotated relative to the rotational member (13, 15) when the delivery coupling (81, 82) is open.

13. The driving and dosing device according to any one of the preceding claims, **characterised in that** the limiting element (20) is permanently - or at least while the dosage is set and while the dosage set is delivered - coupled, rotationally fixed, to a dosage indicating drum (9) and/or the dosage setting element (10).

14. The driving and dosing device according to any one of the preceding claims, **characterised in that** at least one intermediate sleeve (7; 30, 31) is arranged kinematically between the dosage setting element (10) and the limiting element (20), wherein the limiting element (20) engages with the intermediate sleeve (7; 30, 31), such that it is rotationally fixed and can be axially shifted, and is coupled - rotationally fixed - to the dosage setting element (10).

15. The driving and dosing device according to any one of the preceding claims, **characterised in that** in the zero dosage position immediately after a dosage set has been delivered, the limiting element (20) assumes the same position in relation to the piston rod (19) as in the zero dosage position immediately before the dosage is set for said delivery.

16. An injection device for delivering a preferably liquid product, comprising the driving and dosing device according to any one of the preceding claims and a product container (11a) which contains a nominal content amount of the product, wherein only a delivery amount of the nominal content amount can be delivered using the injection device, and a residual amount of the nominal content amount cannot be delivered, wherein the limiting element (20) performs a movement towards the stopping abutment (15a) while the dosage setting element (10) is rotated in the first rotational direction when increasing the dosage, thus reducing a distance - which extends along a helical curve and is directly proportional to the delivery amount - between the stopping abutment (15a) and the limiting element (20), and wherein the limiting element (20) remains stationary in relation to the stopping abutment (15a) while the dosage set is delivered, wherein when the limiting element (20) abuts against the stopping abutment (15a), a dosage which, when delivered, would also cause at least a partial amount of the residual amount to be delivered, or a dosage which would cause less than the residual amount of the nominal content amount to remain in the product container (11a), is prevented from being set.

17. A method for manufacturing an injection device according to Claim 16, wherein: a nominal content amount for a product container (11a) is defined for the injection device; the injection device comprises a stopping abutment (15a) and a limiting element (20); and the limiting element (20) engages with a thread (19a) which predefines a helical curve; **characterised in that** the injection device is assembled such that only a delivery amount of the nominal content amount can be delivered, and a residual amount of the nominal content amount cannot be delivered, wherein this is achieved by arranging the limiting element (20) at a distance - which extends along the helical curve and is directly proportional to the delivery amount - in relation to the stopping abutment (15a).

## Revendications

1. Dispositif d'entraînement et de dosage pour un dispositif d'injection, dans lequel un contenant de produit (11a) est fixé ou peut être fixé au dispositif d'entraînement et de dosage, le dispositif d'entraînement et de dosage comprenant :
a) un boîtier (4, 18),
b) une tige de piston (19), qui présente une extrémité distale (19b), une extrémité proximale (19c) et un filetage (19a) et qui est déplaçable dans une direction de distribution par rapport au boîtier (4, 18) pour une distribution de produit,
c) un organe rotatif (13, 15), lequel est relié de manière opérationnelle, en particulier solidaire en rotation, à une butée d'arrêt (15a) et vient en prise dans le filetage (19a) de la tige de piston (19), dans lequel une rotation de l'organe rotatif (13, 15) par rapport à la tige de piston (19) entraîne le déplacement de la tige de piston (19) par rapport au boîtier (4, 18) et avec son extrémité distale (19b) en avant dans la direction de distribution,
d) un élément de réglage de dose (10), lequel est rotatif dans un premier sens de rotation par rapport au boîtier (4, 18) et/ou à la tige de piston (19) pour l'augmentation d'une dose à distribuer du contenant de produit (11a),
e) un élément de limitation (20), lequel présente une contre-butée (20a) et un filetage (20b), lequel vient en prise dans le filetage (19a) de la tige de piston (19) ou un autre filetage de la tige de piston (19), dans lequel l'élément de réglage de dose (10) est couplé avec l'élément de limitation (20) de telle sorte qu'une rotation de l'élément de réglage de dose (10) par rapport à la tige de piston (19) dans le premier sens de rotation entraîne une rotation de l'élément de limitation (20) par rapport à la tige de piston (19), ce par quoi l'élément de limitation (20) est vissé vers l'extrémité distale (19b) de la tige de piston (19) et la contre-butée (20a) déplacée vers la butée d'arrêt (15a),
f) dans lequel, lorsque la contre-butée (20a) bute contre la butée d'arrêt (15a), l'élément de limitation (20) empêche le réglage d'une dose ou la rotation de l'élément de réglage de dose (10) dans le premier sens de rotation.

2. Dispositif d'entraînement et de dosage selon la revendication 1, **caractérisé en ce que** l'élément de réglage de dose (10) est rotatif dans un deuxième sens de rotation opposé au premier sens de rotation pour la réduction d'une dose réglée et la rotation de l'élément de réglage de dose (10) par rapport à la tige de piston (19) dans le deuxième sens de rotation entraîne une rotation de l'élément de limitation (20) par rapport à la tige de piston (19), ce par quoi l'élément de limitation (20) est vissé vers l'extrémité proximale (19c) de la tige de piston (19) et la contre-butée (20a) éloignée de la butée d'arrêt (15a).

3. Dispositif d'entraînement et de dosage selon l'une quelconque des revendications précédentes, **caractérisé par** un élément d'actionnement (21), dans lequel le dispositif d'entraînement et de dosage est adapté de sorte qu'un actionnement, en particulier la pression, de l'élément d'actionnement (21) par l'utilisateur entraîne la rotation de l'organe rotatif (13, 15) par rapport au boîtier (4, 18) et à la tige de piston (19), ce par quoi la tige de piston (19) se déplace par rapport au boîtier (4, 18) dans la direction de distribution, et le vissage de l'élément de limitation (20) vers l'extrémité proximale (19c) de la tige de piston (19).

4. Dispositif d'entraînement et de dosage selon l'une quelconque des revendications précédentes, **caractérisé en ce qu'**un couplage unidirectionnel (71, 72, 73, 74) est agencé entre le boîtier (4, 18) et l'organe rotatif (13, 15), lequel autorise la rotation de l'organe rotatif (13, 15) dans un sens de rotation qui entraîne le déplacement de la tige de piston (19) dans le sens de distribution et ne l'autorise pas dans le sens de rotation opposé.

5. Dispositif d'entraînement et de dosage selon la revendication précédente, **caractérisé en ce que** le couplage unidirectionnel (71, 72, 73, 74) peut être commuté entre un état de commutation activé et un état de commutation désactivé, dans lequel le couplage unidirectionnel (71, 72, 73, 74) autorise dans son état de commutation activé, la rotation de l'organe rotatif (13, 15) dans un sens de rotation qui entraîne le déplacement de la tige de piston (19) dans le sens de distribution et ne l'autorise pas dans le sens de rotation opposé, et autorise dans son état de commutation désactivé, la rotation de l'organe rotatif (13, 15) dans le sens de rotation opposé.

6. Dispositif d'entraînement et de dosage selon la revendication précédente, **caractérisé par** un support de contenant de produit (11), dans lequel le contenant de produit (11a) peut être reçu ou est reçu et qui est fixé ou peut être fixé de manière amovible à l'extrémité distale du boîtier (4, 18), dans lequel un organe de commutation (12) est agencé entre le support de contenant de produit (11) ou le contenant de produit (11a) et le couplage unidirectionnel (71, 72, 73, 74), lequel peut coulisser par rapport au boîtier (4, 18), en particulier dans la direction proximale, par la fixation du support de contenant de produit (11) ou du contenant de produit (11a) et commute le couplage unidirectionnel (71, 72, 73, 74) dans son état de commutation activé et peut coulisser par désolidarisation du support de contenant de produit (11) ou du contenant de produit (11a), en particulier dans la direction distale, et commute le couplage unidirectionnel (71, 72, 73, 74) dans son état de commutation désactivé.

7. Dispositif d'entraînement et de dosage selon l'une quelconque des deux revendications précédentes, **caractérisé en ce que** le pas de filetage des filetages en prise (19a, 13a) de l'organe rotatif (13, 15) et de la tige de piston (19) est aussi grand qu'aucun auto-blocage n'apparaît, lorsque la tige de piston (19) est reculée dans le sens opposé à la direction de distribution lorsque le couplage unidirectionnel (71, 72, 73, 74) est désactivé, ce par quoi l'organe rotatif (13, 15) réalise une rotation dans le sens de rotation opposé.

8. Dispositif d'entraînement et de dosage selon l'une quelconque des revendications précédentes, **caractérisé par** une douille d'affichage de dose (9), qui est couplée au moins solidaire en rotation, de préférence aussi de manière axialement fixe, avec l'élément de réglage de dose (10), dans lequel la douille d'affichage de dose (9) présente sur sa périphérie une échelle de dose (9b) agencée de manière hélicoïdale, dans lequel le boîtier (4, 18) présente un dispositif d'affichage (18a), en particulier une fenêtre, dans lequel la valeur de l'échelle de dose (9b), qui correspond à la dose réglée, peut être relevée, dans lequel le tambour d'affichage de dose (9) réalise un mouvement de vissage par rapport au boîtier (4, 18) par rotation de l'élément de réglage de dose (10), de sorte que l'échelle de dose (9b) se déplace le long du dispositif d'affichage (18a).

9. Dispositif d'entraînement et de dosage selon la revendication précédente, **caractérisé en ce que** le tambour d'affichage de dose (9) peut être vissé entre une position de dose nulle et une position de dose maximum, dans lequel une butée de dose nulle (4c ; 7d) dans la position de dose nulle du tambour d'affichage de dose (9) empêche la rotation du tambour d'affichage de dose (9) dans le sens de rotation, qui entraîne une réduction de la dose, et l'autorise dans le sens de rotation, qui entraîne une augmentation de la dose, et une butée de dose maximum (7e) dans la position de dose maximum du tambour d'affichage de dose (9) empêche la rotation du tambour d'affichage de dose (9) dans le sens de rotation, qui entraîne une augmentation de la dose, et l'autorise dans le sens de rotation, qui entraîne une réduction de la dose.

10. Dispositif d'entraînement et de dosage selon la revendication précédente, **caractérisé en ce que** la contre-butée (20a) de l'élément de limitation (20) bute contre la butée d'arrêt (15a) et l'élément de limitation (20) empêche la rotation du tambour d'affichage de dose (9) dans le sens de rotation, qui entraîne une augmentation de la dose, lorsque le tambour d'affichage de dose (9) n'est pas dans sa position de dose maximum et/ou soit lorsque la quantité du produit dans le contenant de produit (11a) est inférieure à la dose dans la position de dose maximum soit la partie restante contenue dans le contenant de produit (11a) de la quantité de distribution est inférieure à la dose dans la position de dose maximum.

11. Dispositif d'entraînement et de dosage selon l'une quelconque des revendications précédentes, **caractérisé en ce que** l'élément de limitation (20) est couplé solidaire en rotation avec l'élément de réglage de dose (10), ce par quoi l'élément de limitation (20) est vissé pendant le réglage de la dose et l'administration de la dose réglée le long de la tige de piston (19).

12. Dispositif d'entraînement et de dosage selon l'une quelconque des revendications précédentes, **caractérisé par** un élément d'actionnement (21) actionnable pour la distribution de la dose réglée et un couplage de distribution (81, 82), dans lequel le couplage de distribution (81, 82) est fermé par actionnement, en particulier pression, de l'élément d'actionnement (21) par l'utilisateur du dispositif et ouvert par relâchement de l'élément d'actionnement (21), dans lequel l'organe rotatif (13, 15) est couplé solidaire en rotation avec un tambour d'affichage de dose (9) et/ou avec l'élément de réglage de dose (10), lorsque le couplage de distribution (81, 82) est fermé, et le tambour d'affichage de dose (9) et/ou l'élément de réglage de dose (10) est rotatif par rapport à l'organe rotatif (13, 15), lorsque le couplage de distribution (81, 82) est ouvert.

13. Dispositif d'entraînement et de dosage selon l'une quelconque des revendications précédentes, **caractérisé en ce que** l'élément de limitation (20) est couplé de manière permanente ou au moins pendant le réglage de la dose et la distribution de la dose réglée de manière solidaire en rotation avec un tambour d'affichage de dose (9) et/ou avec l'élément de réglage de dose (10).

14. Dispositif d'entraînement et de dosage selon l'une quelconque des revendications précédentes, **caractérisé en ce qu'**au moins une douille intermédiaire (7 ; 30 ; 31) est agencée cinématiquement entre l'élément de réglage de dose (10) et l'élément de limitation (20), dans laquelle l'élément de limitation (20) vient en prise de manière solidaire en rotation et axialement coulissante et qui est couplée solidaire en rotation avec l'élément de réglage de dose (10).

15. Dispositif d'entraînement et de dosage selon l'une quelconque des revendications précédentes, **caractérisé en ce que** l'élément de limitation (20) dans la position de dose nulle adopte la même position immédiatement après une distribution d'une dose réglée par rapport à la tige de piston (19) que dans la position de dose nulle immédiatement avant le réglage de la dose pour cette distribution.

16. Dispositif d'injection pour la distribution d'un produit de préférence fluide, comprenant le dispositif d'entraînement et de dosage selon l'une quelconque des revendications précédentes et un contenant de produit (11a), qui contient une quantité de remplissage nominale du produit, dans lequel avec le dispositif d'injection seule une quantité de distribution de la quantité de remplissage nominale peut être distribuée et une quantité restante de la quantité de remplissage nominale ne peut pas être distribuée,
g) dans lequel l'élément de limitation (20) réalise un déplacement vers la butée d'arrêt (15a) pendant une rotation de l'élément de réglage de dose (10) dans le premier sens de rotation lors de l'augmentation de la dose, ce par quoi une distance s'étendant le long d'une courbe en forme d'hélice, qui est directement proportionnelle à la quantité de distribution, est réduite entre la butée d'arrêt (15a) et l'élément de limitation (20), et dans lequel l'élément de limitation (20) est à l'arrêt par rapport à la butée d'arrêt (15a) pendant la distribution de la dose réglée, dans lequel lorsque l'élément de limitation (20) bute contre la butée d'arrêt (15a), le réglage d'une dose dont la distribution entraînerait aussi la distribution d'au moins une quantité partielle de la quantité restante est empêché ou ce par quoi le réglage d'une dose, qui entraînerait qu'il reste moins que la quantité restante de la quantité de remplissage nominale dans le contenant de produit (11a), est empêché.

17. Procédé de fabrication d'un dispositif d'injection selon la revendication 16, dans lequel une quantité de remplissage nominale est fixée pour un contenant de produit (11a) pour le dispositif d'injection, le dispositif d'injection présente une butée d'arrêt (15a) et un élément de limitation (20), et l'élément de limitation (20) vient en prise dans un filetage (19a), qui prédéfinit une courbe en forme d'hélice, **caractérisé en ce que** le dispositif d'injection est monté de sorte que seule une quantité de distribution de la quantité de remplissage nominale puisse être distribuée et une quantité restante de la quantité de remplissage nominale ne puisse pas être distribuée, dans lequel ceci a lieu par le fait que l'élément de limitation (20) est agencée par rapport à la butée d'arrêt (15a) avec une distance s'étendant le long de la courbe en forme d'hélice, qui est directement proportionnelle à la quantité de distribution.
